(19) Europäisches Patentamt
European Patent Office
Office européen des brevets

(11) Veröffentlichungsnummer: 0 338 306
A2

(12) # EUROPÄISCHE PATENTANMELDUNG

(21) Anmeldenummer: 89105791.1

(22) Anmeldetag: 03.04.89

(51) Int. Cl.4: C07C 69/734 , C07C 43/257 , C07C 59/68 , C07C 83/00 , C07C 93/14 , C07C 103/00 , C07C 121/75 , C07C 123/00 , C07C 125/065 , C07C 161/02 , C07F 9/40

(30) Priorität: 16.04.88 DE 3812768

(43) Veröffentlichungstag der Anmeldung: 25.10.89 Patentblatt 89/43

(84) Benannte Vertragsstaaten: BE CH DE ES FR GB IT LI NL SE

(71) Anmelder: BAYER AG
D-5090 Leverkusen 1 Bayerwerk(DE)

(72) Erfinder: Kirsten, Rolf, Dr.
Carl-Langhans-Strasse 27
D-4019 Monheim(DE)
Erfinder: Busse, Ulrich, Dr.
Am Bandenfeld 106
D-5657 Haan(DE)
Erfinder: Santel, Hans-Joachim, Dr.
Gruenstrasse 9 a
D-5090 Leverkusen 1(DE)
Erfinder: Schmidt, Robert R., Dr.
Im Waldwinkel 110
D-5060 Bergisch-Gladbach 2(DE)
Erfinder: Strang, Harry, Dr.
Unterdorfstrasse 6 a
D-4000 Düsseldorf 31(DE)

(54) **Halogensubstituierte Phenoxybenzylverbindungen.**

(57) Die Erfindung betrifft neue halogensubstituierte Phenoxybenzylverbindungen der allgemeinen Formel (I)

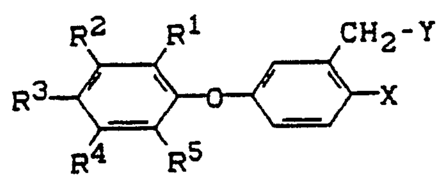

(I)

in welcher
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht,
$R^5$ für Wasserstoff oder Halogen steht,
X für Halogen steht und
Y für Halogen, Cyano, Azido, Thiocyanato, Cyanamino, oder für eine der nachstehenden Gruppierungen

EP 0 338 306 A2

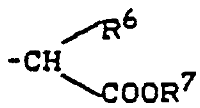, 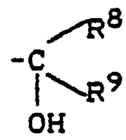, 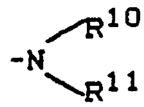, 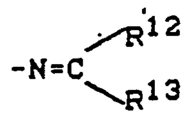,

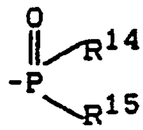, 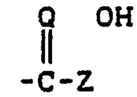 steht,

mehrere Verfahren zu deren Herstellung und deren Verwendung als Herbizide.

## Halogensubstituierte Phenoxybenzylverbindungen

Die Erfindung betrifft neue halogensubstituierte Phenoxybenzylverbindungen, Verfahren zu ihrer Herstellung und ihre Verwendung als Herbizide.

Es ist bereits bekannt, daß bestimmte Phenoxybenzoesäure-Derivate, wie z.B. 3-(2,4-Dichlor-phenoxy)-6-nitro-benzoesäure-methylester (Bifenox) herbizid wirksam sind (vgl. US-P 3 652 645 und US-P 3 776 715). Die Wirkung dieser bekannten Verbindungen ist jedoch nicht immer zufriedenstellend.

Weiter ist bekannt, daß bestimmte halogensubstituierte Phenoxybenzylverbindungen, wie z.B. 2-Chlor-5-(2-chlor-4-trifluormethyl-phenoxy)-benzylchlorid, 2-Chlor-5-(2-chlor-4-trifluormethyl-phenoxy)-benzylcyanid und 2-Brom-5-(2-chlor-4-trifluormethyl-phenoxy)-benzylbromid als Zwischenprodukte zur Herstellung von Herbiziden verwendet werden können (vgl. EP-A 23 890 und EP-A 56 119). Eine herbizide Wirksamkeit dieser Verbindungen ist jedoch nicht aus der Literatur bekannt.

Es wurden nun neue halogensubstituierte Phenoxybenzylverbindungen der allgemeinen Formel (I)

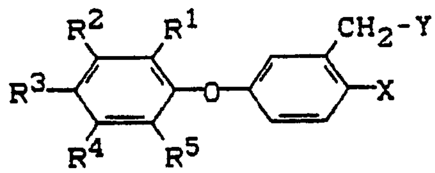

(I)

in welcher
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht,
$R^5$ für Waserstoff oder Halogen steht,
X für Halogen steht und
Y für Halogen, Cyano, Azido, Thiocyanato, Cyanamino, oder für eine der nachstehenden Gruppierungen

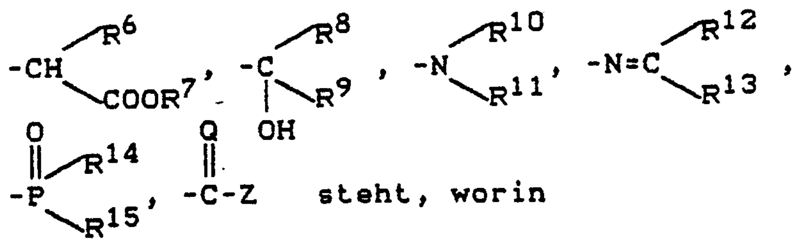

, , , , , steht, worin

$R^6$ für Cyano, Carboxy, gegebenenfalls durch Halogen substituiertes Alkylcarbonyl oder Alkoxycarbonyl steht,
$R^7$ für Wasserstoff oder Alkyl steht,
$R^8$ für Wasserstoff oder Alkyl steht,
$R^9$ für Alkyl, Cycloalkyl, Alkoxycarbonyl oder gegebenenfalls durch Halogen, Alkyl und/oder Alkoxy substituiertes Phenyl steht,
$R^{10}$ für Wasserstoff, für gegebenenfalls durch Halogen, Hydroxy, Alkoxy oder Alkoxycarbonyl substituiertes Alkyl, für Alkenyl, Alkinyl oder Benzyl, für gegebenenfalls durch Halogen oder Alkoxy substituiertes Alkylcarbonyl, für Alkoxycarbonyl, Benzyloxycarbonyl oder Phenoxycarbonyl, für gegebenenfalls durch Halogen substituiertes Alkylsulfonyl, für jeweils gegebenenfalls durch Halogen, Nitro, Alkyl, Halogenalkyl, Alkoxy, Halogenalkoxy, Alkylthio, Alkylsulfinyl, Alkylsulfonyl, Dialkylaminosulfonyl und/oder Alkoxycarbonyl substituiertes Phenylsulfonyl oder Benzylsulfonyl steht,
$R^{11}$ für Hydroxy, für gegebenenfalls durch Alkoxycarbonyl substituiertes Alkoxy, für Alkenyloxy, Benzyloxy, für jeweils gegebenenfalls durch Halogen, Cyano, Nitro, Alkyl, Halogenalkyl, Alkoxy, Halogenalkoxy, Alkylthio, Halo genalkylthio, Alkylsulfinyl, Alkylsulfonyl, Dialkylaminosulfonyl, Alkoxycarbonyl und/oder Alkylendioxy substituiertes Phenyl oder Naphthyl steht, oder $R^{11}$ weiterhin eine der oben für $R^{10}$ angegebenen Bedeutungen hat oder

$R^{11}$ zusammen mit $R^{10}$ für gegebenenfalls verzweigtes und gegebenenfalls durch Sauerstoff unterbrochenes Alkandiyl steht,

$R^{12}$ für Wasserstoff oder Alkyl steht,

$R^{13}$ für Alkyl, Cycloalkyl, Aralkyl, Alkoxy, Dialkylamino oder für gegebenenfalls durch Halogen, Alkyl und/oder Alkoxy substituiertes Phenyl steht

$R^{14}$ für jeweils gegebenenfalls durch Halogen substituiertes Alkyl oder Alkoxy steht,

$R^{15}$ für jeweils gegebenenfalls durch Halogen substituiertes Alkyl oder Alkoxy steht,

Q für Sauerstoff, Schwefel oder NH steht und

Z für Halogen, Cyano, Azido, Thiocyanato, Cyanamino, Hydrazino, Alkylhydrazino, Benzylhydrazino, gegebenenfalls durch Halogen, Alkyl, Halogenalkyl und/oder Alkoxy substituiertes Phenylhydrazino, für Alkylcarbonylhydrazino oder Alkoxycarbonylhydrazino, für gegebenenfalls durch Halogen, Nitro und/oder Alkyl substitu iertes Benzoylhydrazino, für gegebenenfalls durch Halogen substituiertes Alkylsulfonylhydrazino, für gegebenenfalls durch Halogen, Cyano, Nitro, Alkyl, Halogenalkyl, Alkoxy, Halogenalkoxy, Alkylthio, Alkylsulfinyl, Alkylsulfonyl, Dialkylaminosulfonyl und/oder Alkoxycarbonyl substituiertes Phenylsulfonylhydrazino, für Alkylthio, Benzylthio, gegebenenfalls durch Halogen, Alkyl und/oder Alkoxy substituiertes Phenylthio, für Naphthylthio oder Alkoxycarbonylalkylthio oder für eine der nachstehenden Gruppierungen

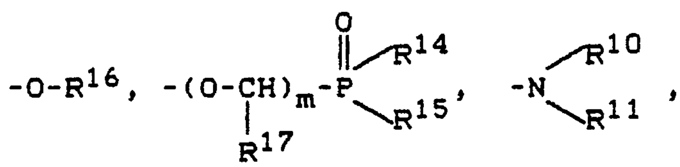

$-O-(CH_2)_n-R^{18}$ steht, worin

m für die Zahlen 0 oder 1 steht,

n für die Zahlen 0, 1, oder 2 steht,

$R^{16}$ für Wasserstoff, für gegebenenfalls durch Halogen, Alkoxy, Alkylthio, Alkylsulfinyl, Alkylsulfonyl, Aralkoxy, Aralkylthio, Alkoxycarbonyl, Alkylaminocarbonyl, Azolyl oder Trialkylsilyl substituiertes Alkyl, für jeweils gegebenenfalls durch Halogen substituiertes Alkenyl oder Alkinyl, für jeweils gegebenenfalls durch Halogen und/oder Alkyl substituiertes Cycloalkyl oder Cycloalkylalkyl, für gegebenenfalls durch Halogen, Alkyl, Alkoxy und/oder Alkoxycarbonyl substituiertes Benzyl, für jeweils gegebenenfalls durch Halogen, Alkyl, Halogenalkyl, Alkoxy und/oder Halogenalkoxy substituiertes Phenyl oder Naphthyl, oder für ein Ammonium-, Alkylammonium-, Alkalimetall- oder Erdalkalimetall-Äquivalent steht,

$R^{17}$ für Wasserstoff, Alkyl, jeweils gegebenenfalls durch Halogen, Alkyl, Halogenalkyl und/oder Alkoxy substituiertes Phenyl, Furyl, Thienyl oder Pyridyl steht,

$R^{18}$ für eine gegebenenfalls durch Halogen und/oder Alkyl substituierte heterocyclische Gruppierung aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht, sowie

$R^{10}$, $R^{11}$, $R^{14}$ und $R^{15}$ die oben angegebenen Bedeutungen haben,

sowie Salze von Verbindungen der Formel (I) gefunden, wobei die als Zwischenprodukte bekannten Verbindungen 2-Chlor-5-(2-chlor-4-trifluormethyl-phenoxy)-benzylchlorid und 2-Chlor-5-(2-chlor-4-trifluormethylphenoxy)-benzylcyanid (vgl. EP-A 23 890, S. 12) und 2-Brom-5-(2-chlor-4-trifluormethyl-phenoxy)-benzylbromid (vgl. EP-A 56 119, S. 22) ausgenommen sind.

Weiter wurde gefunden, daß man die neuen halogensubstituierten Phenoxybenzylverbindungen der allgemeinen Formel (I) sowie deren Salze erhält, wenn man

(a) halogensubstituierte Phenoxytoluol-Derivate der allgemeinen Formel (II)

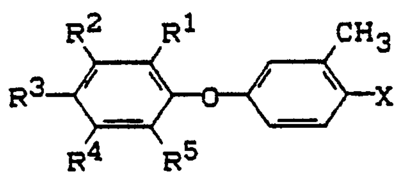

(II)

in welcher

$R^1$, $R^2$, $R^3$, $R^4$, $R^5$ und X die oben angegebenen Bedeutungen haben,

mit Halogenierungsmitteln, gegebenenfalls unter Belichtung, gegebenenfalls in Gegenwart von Katalysatoren und gegebenenfalls in Gegenwart von Verdünnungsmitteln umsetzt, oder wenn man

(b) halogensubstituierte Phenoxybenzylhalogenide der allgemeinen Formel (Ia)

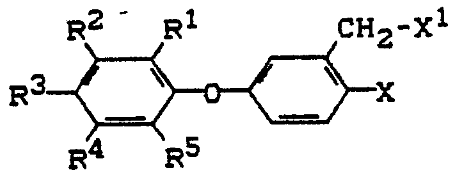

(Ia)

in welcher

$R^1$, $R^2$, $R^3$, $R^4$, $R^5$ und X die oben angegebenen Bedeutungen haben und

$X^1$ für Halogen steht,

mit nucleophilen Verbindungen der allgemeinen Formel (III)

M - Y (III)

in welcher

Y die oben angegebene Bedeutung hat und

M für Wasserstoff oder ein Metalläquivalent steht,

gegebenenfalls in Gegenwart eines Säureakzeptors und gegebenenfalls in Gegenwart eines Verdünnungsmittels umsetzt, oder wenn man

(c) halogensubstituierte Phenoxybenzylhalogenide der allgemeinen Formel (Ia)

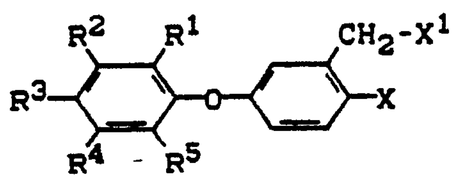

(Ia)

in welcher

$R^1$, $R^2$, $R^3$, $R^4$, $R^5$ und X die oben angegebenen Bedeutungen haben und

$X^1$ für Halogen steht,

mit Alkoxy-Phosphorverbindungen der allgemeinen Formel (IV)

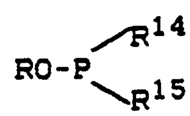

(IV)

in welcher

$R^{14}$ und $R^{15}$ die oben angegebenen Bedeutungen haben und

R für Niederalkyl steht,

gegebenenfalls in Gegenwart von Verdünnungsmitteln umsetzt, oder wenn man

(d) halogensubstituierte Phenoxybenzylcyanide der allgemeinen Formel (Ib)

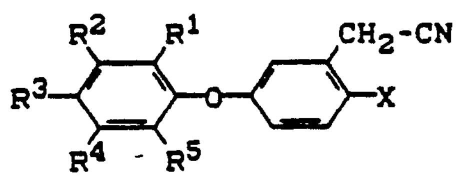

(Ib)

in welcher

$R^1$, $R^2$, $R^3$, $R^4$, $R^5$ und X die oben angegebenen Bedeutungen haben,

mit nucleophilen Verbindungen der allgemeinen Formel (V)

M' - Z (V)
in welcher
Z die oben angegebene Bedeutung hat und
M' für Wasserstoff oder ein Metalläquivalent steht,
gegebenenfalls in Gegenwart eines Katalysators und gegebenenfalls in Gegenwart eines Verdünnungsmittels umsetzt, oder wenn man

(e) halogensubstituierte Phenoxybenzylverbindungen der allgemeinen Formel (Ic)

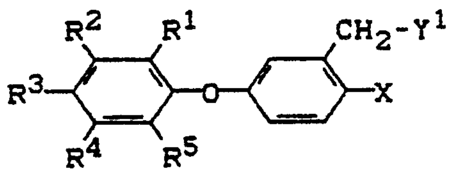
(Ic)

in welcher
$R^1$, $R^2$, $R^3$, $R^4$, $R^5$ und X die oben angegebenen Bedeutungen haben und
$Y^1$ für Cyano oder Alkoxycarbonyl steht,
mit Wasser, gegebenenfalls in Gegenwart eines Verseifungshilfsmittels und gegebenenfalls in Gegenwart eines organischen Lösungsmittels, umsetzt, oder wenn man

(f) halogensubstituierte Phenoxyphenylessigsäuren der allgemeinen Formel (Id)

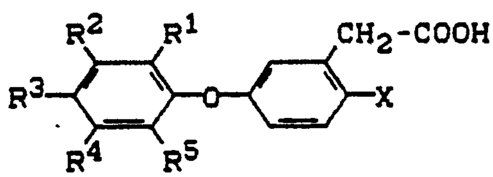
(Id)

in welcher
$R^1$, $R^2$, $R^3$, $R^4$, $R^5$ und X die oben angegebenen Bedeutungen haben,
mit Säurehalogeniden, gegebenenfalls in Gegenwart von Katalysatoren und gegebenenfalls in Gegenwart von Verdünnungsmitteln, umsetzt, oder wenn man

(g) halogensubstituierte Phenoxyphenylessigsäurehalogenide der allgemeinen Formel (Ie)

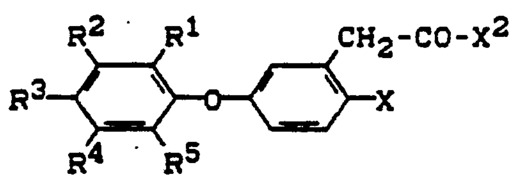
(Ie)

in welcher
$R^1$, $R^2$, $R^3$, $R^4$, $R^5$ und X die oben angegebenen Bedeutungen haben und
$X^2$ für Halogen steht
mit nucleophilen Verbindungen der allgemeinen Formel (V)
M' - Z (V)
in welcher
Z die angegebene Bedeutung hat und
M' für Wasserstoff oder ein Metalläquivalent steht,
gegebenenfalls in Gegenwart eines Säureakzeptors und gegebenenfalls in Gegenwart eines Verdünnungsmittels umsetzt, oder wenn man

(h) halogensubstituierte Phenoxybenzylhalogenide der allgemeinen Formel (Ia)

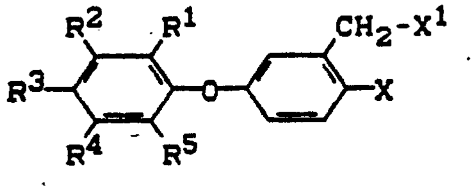 (Ia)

in welcher
$R^1$, $R^2$, $R^3$, $R^4$, $R^5$ und X die oben angegebenen Bedeutungen haben und
$X^1$ für Halogen steht,
mit Metallierungsmitteln und anschließend mit Carbonylverbindungen der allgemeinen Formel (VI)

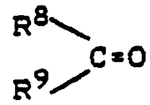 (VI)

in welcher
$R^8$ und $R^9$ die oben angegebenen Bedeutungen haben,
gegebenenfalls in Gegenwart eines Verdünnungsmittels umsetzt, oder wenn man
(i) Phenoxybenzylverbindungen der allgemeinen Formel (VII)

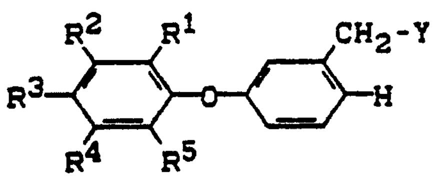 (VII)

in welcher
$R^1$, $R^2$, $R^3$, $R^4$, $R^5$ und Y die oben angegebenen Bedeutungen haben,
mit Halogenen, gegebenenfalls in Gegenwart von Katalysatoren und gegebenenfalls in Gegenwart von Verdünnungsmitteln umsetzt und gegebenenfalls anschließend aus den Verbindungen der Formel (I) durch Umsetzung mit Säuren oder Basen Salze herstellt.

Schließlich wurde gefunden, daß die neuen halogensubstituierten Phenoxybenzylverbindungen der Formel (I) hervorragende herbizide Eigenschaften aufweisen.

Überraschenderweise sind die erfindungsgemäßen halogensubstituierten Phenoxybenzylverbindungen der Formel (I) gegen Unkräuter wesentlich stärker wirksam als 3-(2,4-Dichlor-phenoxy)-6-nitro-benzoesäuremethylester, welches ein strukturell ähnlicher vorbekannter Wirkstoff gleicher Wirkungsrichtung ist.

Die einzelnen Kohlenwasserstoffketten wie z.B. Alkyl, Alkoxy, Alkylthio, Alkoxycarbonylalkyl, Alkenyl, Alkinyl, Alkylsulfonyl sind jeweils geradkettig oder verzweigt. Im Falle von substituierten Resten kann die Substitution jeweils einfach oder mehrfach, gleich oder verschieden erfolgen.

Die Erfindung betrifft vorzugsweise Verbindungen der Formel (I), in welcher
$R^1$ für Wasserstoff, Fluor, Chlor, Brom, Cyano oder Trifluormethyl steht,
$R^2$ für Wasserstoff, Fluor, Chlor oder Brom steht,
$R^3$ für Fluor, Chlor, Brom, Cyano, Trifluormethyl, Trifluormethoxy oder Trifluormethylsulfonyl steht,
$R^4$ für Wasserstoff, Fluor, Chlor oder Brom steht,
$R^5$ für Wasserstoff, Fluor, Chlor oder Brom steht,
X für Fluor, Chlor oder Brom steht und
Y für Fluor, Chlor, Brom, Iod, Cyano, Azido, Thiocyanato, Cyanamino oder für eine der nachstehenden Gruppierungen

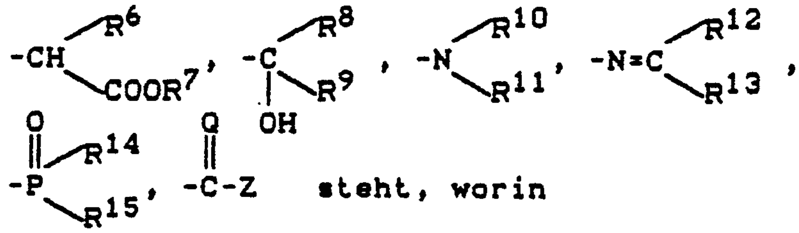

steht, worin

$R^6$ für Cyano, Carboxy, gegebenenfalls durch Fluor und/oder Chlor substituiertes $C_1$-$C_4$-Alkyl-carbonyl oder $C_1$-$C_4$-Alkoxy-carbonyl steht,

$R^7$ für Wasserstoff oder $C_1$-$C_4$-Alkyl steht,

$R^8$ für Wasserstoff oder $C_1$-$C_4$-Alkyl steht,

$R^9$ für $C_1$-$C_4$-Alkyl, $C_3$-$C_6$-Cycloalkyl, $C_1$-$C_4$-Alkoxy-carbonyl oder gegebenenfalls durch Fluor, Chlor, Brom, $C_1$-$C_4$-Alkyl und/oder $C_1$-$C_4$-Alkoxy substituiertes Phenyl steht,

$R^{10}$ für Wasserstoff, gegebenenfalls durch Fluor, Chlor, Hydroxy, $C_1$-$C_4$-Alkoxy oder $C_1$-$C_4$-Alk oxy-carbonyl substituiertes $C_1$-$C_6$-Alkyl, für $C_3$-$C_6$-Alkenyl, $C_3$-$C_6$-Alkinyl oder Benzyl, für gegebenenfalls durch Fluor, Chlor, Brom oder $C_1$-$C_4$-Alkoxy substituiertes $C_1$-$C_4$-Alkyl-carbonyl, für $C_1$-$C_4$-Alkoxy-carbonyl, Benzyloxycarbonyl oder Phenoxycarbonyl, für gegebenenfalls durch Fluor und/oder Chlor substituiertes $C_1$-$C_6$-Alkylsulfonyl, für jeweils gegebenenfalls durch Fluor, Chlor, Brom, Nitro, Methyl, Trifluormethyl, $C_1$-$C_2$-Alkoxy, durch Fluor und/oder Chlor substituiertes $C_1$-$C_2$-Alkoxy, $C_1$-$C_2$-Alkylthio, $C_1$-$C_2$-Alkylsulfinyl, $C_1$-$C_2$-Alkylsulfonyl, Di-($C_1$-$C_2$-alkyl)-aminosulfonyl und/oder $C_1$-$C_2$-Alkoxy-carbonyl substituiertes Phenylsulfonyl oder Benzylsulfonyl steht,

$R^{11}$ für Hydroxy, für gegebenenfalls durch $C_1$-$C_6$-Alkoxy-carbonyl substituiertes $C_1$-$C_6$-Alkoxy, für $C_3$-$C_6$-Alkenyloxy oder Benzyloxy, für jeweils gegebenenfalls durch Fluor, Chlor, Brom, Cyano, Nitro, $C_1$-$C_4$-Alkyl, Trifluormethyl, $C_1$-$C_4$-Alkoxy, durch Fluor und/oder Chlor substituiertes $C_1$-$C_2$-Alkoxy, $C_1$-$C_4$-Alkylthio, durch Fluor und/oder Chlor substituiertes $C_1$-$C_4$-Alkylthio, $C_1$-$C_4$-Alkylsulfinyl, $C_1$-$C_4$-Alkylsulfonyl, Di-($C_1$-$C_2$-alkyl)-aminosulfonyl, $C_1$-$C_4$-Alkoxy-carbonyl und/oder $C_1$-$C_2$-Alkylendioxy substituiertes Phenyl oder Naphthyl steht, oder $R^{11}$ weiterhin eine der oben für $R^{10}$ vorzugsweise angegebenen Bedeutungen hat oder $R^{11}$ zusammen mit $R^{10}$ für gegebenenfalls verzweig tes und gegebenenfalls durch Sauerstoff unterbrochenes $C_2$-$C_6$-Alkandiyl steht,

$R^{12}$ für Wasserstoff oder $C_1$-$C_4$-Alkyl steht,

$R^{13}$ für $C_1$-$C_4$-Alkyl, $C_3$-$C_6$-Cycloalkyl, Benzyl, $C_1$-$C_4$-Alkoxy, Di-($C_1$-$C_2$-alkyl)-amino oder für gegebenenfalls durch Fluor, Chlor, Brom, $C_1$-$C_4$-Alkyl und/oder $C_1$-$C_4$-Alkoxy substituiertes Phenyl steht,

$R^{14}$ für jeweils gegebenenfalls durch Fluor und/oder Chlor substituiertes $C_1$-$C_4$-Alkyl oder $C_1$-$C_4$-Alkoxy steht,

$R^{15}$ für jeweils gegebenenfalls durch Fluor und/oder Chlor substituiertes $C_1$-$C_4$-Alkyl oder $C_1$-$C_4$-Alkoxy steht,

Q für Sauerstoff, Schwefel oder NH steht und

Z für Chlor, Cyano, Azido, Thiocyanato, Cyanamino, Hydrazino, $C_1$-$C_4$-Alkylhydrazino, Benzylhydrazino, gegebenenfalls durch Fluor, Chlor, Brom, $C_1$-$C_4$-Alkyl, Trifluormethyl und/oder $C_1$-$C_4$-Alkoxy substituiertes Phenylhydrazino, für $C_1$-$C_4$-Alkyl-carbonylhydrazino oder $C_1$-$C_4$-Alkoxy-carbonylhydrazino, für gegebenenfalls durch Fluor, Chlor, Brom, Nitro und/oder $C_1$-$C_4$-Alkyl substituiertes Benzoylhydrazino, für gegebenenfalls durch Fluor und/oder Chlor substituiertes $C_1$-$C_4$-Alkylsulfonylhydrazino, für gegebenenfalls durch Fluor, Chlor, Brom, Cyano, Nitro, Methyl, Trifluormethyl, $C_1$-$C_2$-Alkoxy, durch Fluor und/oder Chlor substituiertes $C_1$-$C_2$-Alkoxy, $C_1$-$C_2$-Alkylthio, $C_1$-$C_2$-Alkylsulfinyl, $C_1$-$C_2$-Alkylsulfonyl, Di-($C_1$-$C_2$-alkyl)-aminosulfonyl und/oder $C_1$-$C_2$-Alkoxycarbonyl substituiertes Phenylsulfonylhydrazino, für $C_1$-$C_4$-Alkylthio, Benzylthio, gegebenenfalls durch Fluor, Chlor, Brom, $C_1$-$C_4$-Alkyl und/oder $C_1$-$C_4$-Alkoxy substituiertes Phenylthio, für Naphthylthio oder $C_1$-$C_4$-Alkoxy-carbonyl-$C_1$-$C_2$-alkylthio oder für eine der nachstehenden Gruppierungen

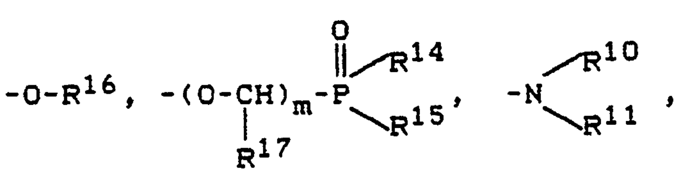

$-O-(CH_2)_n-R^{18}$ steht, worin

m für die Zahlen 0 oder 1 steht,
n für die Zahlen 0, 1 oder 2 steht,
$R^{16}$ für Wasserstoff, für gegebenenfalls durch Fluor, Chlor, $C_1$-$C_4$-Alkoxy, $C_1$-$C_4$-Alkylthio, $C_1$-$C_4$-Alkylsulfinyl, $C_1$-$C_4$-Alkylsulfonyl , Benzyloxy, Benzylthio, $C_1$-$C_4$-Alkoxy-carbonyl, $C_1$-$C_4$-Alkylamino-carbonyl, Pyrazolyl oder Trimethylsilyl substituiertes $C_1$-$C_6$-Alkyl, für jeweils gegebenenfalls durch Fluor und/oder Chlor substituiertes $C_3$-$C_4$-Alkenyl oder $C_3$-$C_4$-Alkinyl, für jeweils gegebenenfalls durch Fluor, Chlor und/oder $C_1$-$C_4$-Alkyl substituiertes $C_3$-$C_6$-Cycloalkyl oder $C_3$-$C_6$-Cycloalkyl-$C_1$-$C_3$-alkyl, für gegebenenfalls durch Fluor, Chlor, Brom, $C_1$-$C_4$-Alkyl, $C_1$-$C_4$-Alkoxy und/oder $C_1$-$C_4$-Alkoxy-carbonyl substituiertes Benzyl, für jeweils gegebenenfalls durch Fluor, Chlor, Brom, $C_1$-$C_4$-Alkyl, Trifluormethyl, $C_1$-$C_4$-Alkoxy und/oder durch Fluor und/oder Chlor substituiertes $C_1$-$C_2$-Alkoxy substituiertes Phenyl oder Naphthyl, oder für ein Ammonium-, $C_1$-$C_4$-Alkylammonium-, Natrium-, Kalium- oder Calcium-äquivalent steht,
$R^{17}$ für Wasserstoff, $C_1$-$C_4$-Alkyl, jeweils gegebenenfalls durch Fluor, Chlor, Brom, $C_1$-$C_4$-Alkyl, Trifluormethyl, und/oder $C_1$-$C_4$-Alkoxy substituiertes Phenyl, Furyl, Thienyl oder Pyridyl steht,
$R^{18}$ für eine gegebenenfalls durch Fluor, Chlor, Brom und/oder $C_1$-$C_4$-Alkyl substituierte heterocyclische Gruppierung aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl, oder Pyrimidinyl steht, sowie
$R^{10}$, $R^{11}$, $R^{14}$, und $R^{15}$ die oben vorzugsweise angegebenen Bedeutungen haben,
mit Ausnahme der oben durch einen Disclaimer ausgenommenen Verbindungen.

Eine Gruppe ganz besonders bevorzugter Verbindungen der Formel (I) sind diejenigen, in welcher
$R^1$ für Wasserstoff, Fluor oder Chlor steht,
$R^2$ für Wasserstoff, Fluor oder Chlor steht,
$R^3$ für Trifluormethyl steht,
$R^4$ für Wasserstoff, Fluor oder Chlor steht,
$R^5$ für Wasserstoff, Fluor oder Chlor steht,
X für Chlor oder Brom steht und
Y für Chlor, Brom oder Cyano steht
mit Ausnahme der oben durch einen Disclaimer ausgenommenen Verbindungen.

Eine andere Gruppe ganz besonders bevorzugter Verbindungen der Formel (I) sind diejenigen, in welcher
$R^1$ für Wasserstoff, Fluor oder Chlor steht,
$R^2$ für Wasserstoff, Fluor oder Chlor steht,
$R^3$ für Trifluormethyl steht,
$R^4$ für Wasserstoff, Fluor oder Chlor steht,
$R^5$ für Wasserstoff, Fluor oder Chlor steht,
X für Chlor oder Brom steht und
Y für Thiocyanato, Cyanamino oder für eine der nachstehenden Gruppierungen

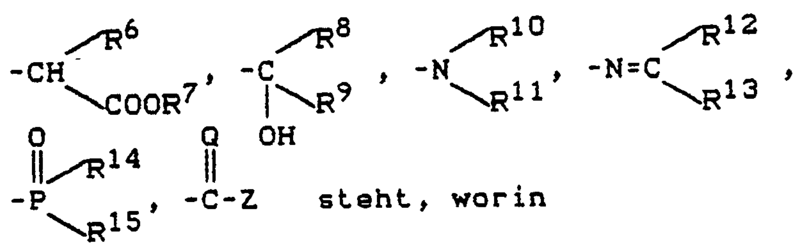

steht, worin

$R^6$ für Carboxy, Acetyl, Methoxycarbonyl oder Ethoxycarbonyl steht,
$R^7$ für Wasserstoff, Methyl oder Ethyl steht,
$R^8$ für Methyl steht,
$R^9$ für Methyl, Ethyl, Cyclopropyl, Methoxycarbonyl, Ethoxycarbonyl oder Phenyl steht,
$R^{10}$ für Wasserstoff, $C_1$-$C_4$-Alkyl, $C_1$-$C_2$-Alkoxycarbonyl-$C_1$-$C_2$-alkyl, $C_3$-$C_4$-Alkenyl, $C_3$-$C_4$-Alkinyl oder Benzyl, für gegebenenfalls durch Fluor und/oder Chlor substituiertes $C_1$-$C_4$-Alkyl-carbonyl, für $C_1$-$C_4$-Alkoxy-carbonyl, Benzyloxycarbonyl oder Phenoxycarbonyl, für gegebenenfalls durch Fluor und/oder Chlor substituiertes $C_1$-$C_4$-Alkylsulfonyl, für jeweils gegebenenfalls durch Fluor, Chlor, Brom, Nitro, Methyl, Trifluormethyl, Methoxy, Difluormethoxy und/ oder Methoxycarbonyl substituiertes Phenylsulfonyl oder Benzylsulfonyl steht,
$R^{11}$ für Hydroxy, für gegebenenfalls durch $C_1$-$C_4$-Alkoxy-carbonyl substituiertes $C_1$-$C_4$-Alkoxy, Allyloxy oder

Benzyloxy, für jeweils gegebenenfalls durch Fluor, Chlor, Brom, Methyl, Ethyl, Trifluormethyl, Methoxy, Ethoxy, Difluormethoxy, Methylthio, Ethylthio, Trifluormethylthio, Methoxycarbonyl, oder Ethoxycarbonyl substituiertes Phenyl oder Naphthyl steht, oder $R^{11}$ weiterhin eine der oben für $R^{10}$ insbesondere angegebenen Bedeutungen hat oder

$R^{11}$ zusammen mit $R^{10}$ für gegebenenfalls verzweigtes und gegebenenfalls durch Sauerstoff unterbrochenes $C_4$-$C_5$-Alkandiyl steht,

$R^{12}$ für Wasserstoff oder Methyl steht,

$R^{13}$ für Dimethylamino steht,

$R^{14}$ für Methoxy, Ethoxy, n- oder iso-Propoxy steht,

$R^{15}$ für Methoxy, Ethoxy, n- oder iso-Propoxy steht,

Q für Sauerstoff oder NH steht und

Z für Chlor, $C_1$-$C_4$-Alkylthio, Benzylthio, $C_1$-$C_2$-Alkoxycarbonyl-$C_1$-$C_2$-alkylthio oder für eine der nachstehenden Gruppierungen

$-O-R^{16}$, 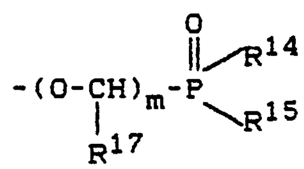, 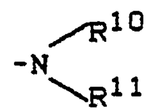,

$-O-(CH_2)_n-R^{18}$ steht, worin

m für die Zahlen 0 oder 1 steht,

n für die Zahlen 0, 1 oder 2 steht,

$R^{16}$ für Wasserstoff, $C_1$-$C_4$-Alkyl, $C_1$-$C_2$-Alkoxy-$C_1$-$C_2$-alkyl, $C_1$-$C_2$-Alkylthio-$C_1$-$C_2$-alkyl, $C_1$-$C_2$-Alkylsulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_2$-Alkoxycarbonyl-$C_1$-$C_2$-alkyl oder $C_1$-$C_2$-Alkylamino-carbonyl-$C_1$-$C_2$-alkyl steht,

$R^{17}$ für Wasserstoff, Methyl, Phenyl, Furyl, Thienyl oder Pyridyl steht,

$R^{18}$ für eine gegebenenfalls durch Chlor und/oder Methyl substituierte heterocyclische Gruppierung aus der Reihe Furyl, Tetrahydrofuryl, Thienyl, Perhydropyranyl, Oxazolyl, Thiazolyl oder Dioxolanyl steht, sowie

$R^{10}$, $R^{11}$, $R^{14}$ und $R^{15}$ die oben insbesondere angegebenen Bedeutungen haben.

Die Erfindung betrifft weiter vorzugsweise Salze

α) aus Verbindungen der Formel (I), in welcher $R^1$, $R^2$, $R^3$, $R^4$, $R^5$, und X die oben angegebenen Bedeutungen haben und Y für Amino, Alkylamino oder Dialkylamino, insbesondere für Amino, $C_1$-$C_6$-Alkylamino oder Di-$C_1$-$C_6$-alkylamino steht,

mit Protonensäuren, wie vorzugsweise Salzsäure, Schwefelsäure, Phosphorsäure, Methansulfonsäure, Benzol- oder p-Toluol-sulfonsäure oder Naphthalinmono- oder -di-sulfonsäuren, oder

β) aus Verbindungen der Formel (I), in welcher $R^1$, $R^2$, $R^3$, $R^4$, $R^5$ und X die oben angegebenen Bedeutungen haben und Y für eine der oben angegebenen Gruppierungen steht, soweit diese -COOH, -CO-NH- oder $-SO_2$-NH- Komponenten enthalten,

mit Basen, wie vorzugsweise Natrium-, Kalium- oder Calcium-hydroxid, -hydrid, -amid oder -carbonat, Natrium- oder Kalium-$C_1$-$C_4$-alkanolaten, Ammoniak, $C_1$-$C_4$-Alkylaminen, Di-($C_1$-$C_4$-alkyl)-aminen oder Tri-($C_1$-$C_4$-alkyl)-aminen.

Beispiele für die erfindungsgemäßen Verbindungen der Formel (I) sind in der nachstehenden Tabelle 1 aufgeführt - vgl. auch die Herstellungsbeispiele.

Tabelle 1: Beispiele für die Verbindungen der Formel (I)

| $R^1$ | $R^2$ | $R^3$ | $R^4$ | $R^5$ | X | Y |
|---|---|---|---|---|---|---|
| Cl | H | $CF_3$ | H | Cl | Cl | Cl |
| Cl | H | $CF_3$ | H | F | Cl | Cl |
| Cl | H | $CF_3$ | Cl | Cl | Cl | Cl |
| Cl | H | $CF_3$ | F | Cl | Cl | Cl |
| Cl | H | $CF_3$ | H | Cl | Br | Br |
| Cl | H | $CF_3$ | H | Cl | Cl | Br |
| Cl | H | $CF_3$ | H | Cl | Br | Cl |
| Cl | H | $CF_3$ | H | F | Br | Br |
| Cl | H | $CF_3$ | H | F | Br | Br |
| Cl | H | $CF_3$ | H | F | Cl | Br |
| Cl | H | $CF_3$ | F | F | Cl | Cl |
| Cl | H | $CF_3$ | H | Cl | Cl | CN |
| Cl | H | $CF_3$ | H | Cl | Br | CN |
| Cl | H | $CF_3$ | H | F | Cl | CN |
| Cl | H | $CF_3$ | H | F | Br | CN |
| Cl | H | $CF_3$ | Cl | Cl | Cl | CN |
| Cl | H | $CF_3$ | H | H | Cl | SCN |
| Cl | H | $CF_3$ | H | H | Br | SCN |
| Cl | H | $CF_3$ | H | Cl | Cl | SCN |
| Cl | H | $CF_3$ | Cl | Cl | Cl | SCN |
| Cl | H | $CF_3$ | F | Cl | Cl | SCN |
| Cl | H | $CF_3$ | H | F | Cl | SCN |

Tabelle 1 - Fortsetzung

| $R^1$ | $R^2$ | $R^3$ | $R^4$ | $R^5$ | X | Y |
|---|---|---|---|---|---|---|
| Cl | H | $CF_3$ | H | Cl | Br | SCN |
| Cl | H | $CF_3$ | H | Cl | Cl | NHCN |
| Cl | H | $CF_3$ | Cl | Cl | Cl | NHCN |
| Cl | H | $CF_3$ | H | F | Cl | NHCN |
| Cl | H | $CF_3$ | H | H | Br | NHCN |
| Cl | H | $CF_3$ | H | H | Cl | $-CH(COOH)_2$ |
| Cl | H | $CF_3$ | H | Cl | Cl | $-CH(COOH)_2$ |
| Cl | H | $CF_3$ | H | F | Cl | $-CH(COOH)_2$ |
| Cl | H | $CF_3$ | Cl | Cl | Cl | $-CH(COOH)_2$ |
| Cl | H | $CF_3$ | F | Cl | Cl | $-CH(COOH)_2$ |
| Cl | H | $CF_3$ | H | Cl | Br | $-CH(COOH)_2$ |
| Cl | H | $CF_3$ | H | H | Cl | $-CH(COOCH_3)_2$ |
| Cl | H | $CF_3$ | H | Cl | Cl | $-CH(COOCH_3)_2$ |
| Cl | H | $CF_3$ | Cl | Cl | Cl | $-CH(COOCH_3)_2$ |
| Cl | H | $CF_3$ | F | Cl | Cl | $-CH(COOCH_3)_2$ |
| Cl | H | $CF_3$ | H | F | Cl | $-CH(COOCH_3)_2$ |
| Cl | H | $CF_3$ | H | H | Br | $-CH(COOCH_3)_2$ |
| Cl | H | $CF_3$ | H | H | Cl | $-CH(COOC_2H_5)_2$ |
| Cl | H | $CF_3$ | H | Cl | Cl | $-CH(COOC_2H_5)_2$ |
| Cl | H | $CF_3$ | Cl | Cl | Cl | $-CH(COOC_2H_5)_2$ |
| Cl | H | $CF_3$ | F | Cl | Cl | $-CH(COOC_2H_5)_2$ |
| Cl | H | $CF_3$ | H | F | Cl | $-CH(COOC_2H_5)_2$ |

Tabelle 1 - Fortsetzung

| $R^1$ | $R^2$ | $R^3$ | $R^4$ | $R^5$ | X | Y |
|---|---|---|---|---|---|---|
| Cl | H | $CF_3$ | H | H | Br | $-CH(COOC_2H_5)_2$ |
| Cl | H | $CF_3$ | H | H | Cl | 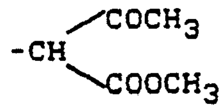 |
| Cl | H | $CF_3$ | H | Cl | Cl | 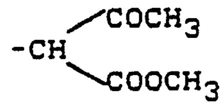 |
| Cl | H | $CF_3$ | H | F | Cl | 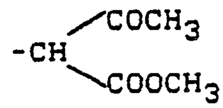 |
| Cl | H | $CF_3$ | H | H | Cl | 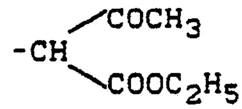 |
| Cl | H | $CF_3$ | H | Cl | Cl | 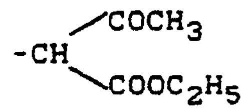 |
| Cl | H | $CF_3$ | H | F | Cl | 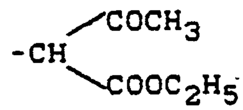 |
| Cl | H | $CF_3$ | H | H | Cl | 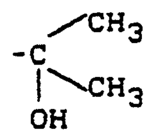 |
| Cl | H | $CF_3$ | H | Cl | Cl | 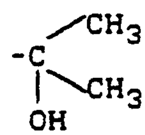 |
| Cl | H | $CF_3$ | H | F | Cl | 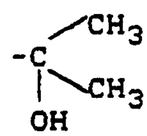 |

Tabelle 1 - Fortsetzung

| $R^1$ | $R^2$ | $R^3$ | $R^4$ | $R^5$ | X | Y |
|---|---|---|---|---|---|---|
| Cl | H | $CF_3$ | H | H | Cl | 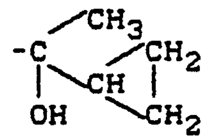 |
| Cl | H | $CF_3$ | H | Cl | Cl | 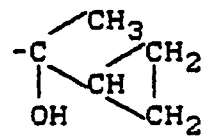 |
| Cl | H | $CF_3$ | H | F | Cl | 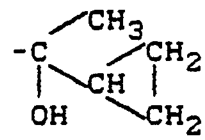 |
| Cl | H | $CF_3$ | H | H | Cl | 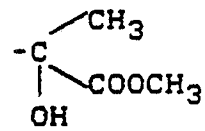 |
| Cl | H | $CF_3$ | H | Cl | Cl | 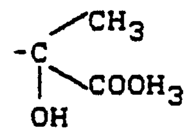 |
| Cl | H | $CF_3$ | H | F | Cl | 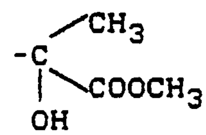 |
| Cl | H | $CF_3$ | H | H | Cl | 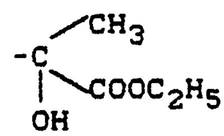 |
| Cl | H | $CF_3$ | H | H | Br | 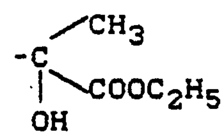 |

Tabelle 1 - Fortsetzung

| $R^1$ | $R^2$ | $R^3$ | $R^4$ | $R^5$ | X | Y |
|---|---|---|---|---|---|---|
| Cl | H | $CF_3$ | H | Cl | Cl | 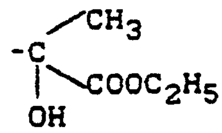 |
| Cl | H | $CF_3$ | H | F | Cl | 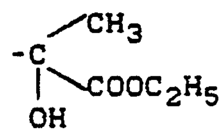 |
| Cl | H | $CF_3$ | H | H | Cl | $NH_2$ |
| Cl | H | $CF_3$ | H | Cl | Cl | $NH_2$ |
| Cl | H | $CF_3$ | H | F | Cl | $NH_2$ |
| Cl | H | $CF_3$ | Cl | Cl | Cl | $NH_2$ |
| Cl | H | $CF_3$ | F | Cl | Cl | $NH_2$ |
| Cl | H | $CF_3$ | H | H | Br | $NH_2$ |
| Cl | H | $CF_3$ | H | Cl | Br | $NH_2$ |
| Cl | H | $CF_3$ | Cl | Cl | Br | $NH_2$ |
| Cl | H | $CF_3$ | F | Cl | Br | $NH_2$ |
| Cl | H | $CF_3$ | H | F | Br | $NH_2$ |
| Cl | H | $CF_3$ | H | H | Cl | $NHCH_3$ |
| Cl | H | $CF_3$ | H | Cl | Cl | $NHCH_3$ |
| Cl | H | $CF_3$ | H | F | Cl | $NHCH_3$ |
| Cl | H | $CF_3$ | H | H | Cl | $NHC_2H_5$ |
| Cl | H | $CF_3$ | H | Cl | Cl | $NHC_2H_5$ |
| Cl | H | $CF_3$ | H | F | Cl | $NHC_2H_5$ |

<u>Tabelle 1</u> - Fortsetzung

| $R^1$ | $R^2$ | $R^3$ | $R^4$ | $R^5$ | X | Y |
|---|---|---|---|---|---|---|
| Cl | H | $CF_3$ | H | H | Cl | $NHC_3H_7$ |
| Cl | H | $CF_3$ | H | Cl | Cl | $NHCH(CH_3)_2$ |
| Cl | H | $CF_3$ | H | F | Cl | $NHCH(CH_3)_2$ |
| Cl | H | $CF_3$ | H | H | Cl | $NHC_4H_9$ |
| Cl | H | $CF_3$ | H | Cl | Cl | $NHCH_2CH(CH_3)_2$ |
| Cl | H | $CF_3$ | H | F | Cl | $NH\text{-}CHCH_2CH_3$<br>\|<br>$CH_3$ |
| Cl | H | $CF_3$ | H | H | Cl | $N(CH_3)_2$ |
| Cl | H | $CF_3$ | H | H | Cl | $N(C_2H_5)_2$ |
| Cl | H | $CF_3$ | H | Cl | Cl | $NHCH_2COOCH_3$ |
| Cl | H | $CF_3$ | H | F | Cl | $NHCH_2COOC_2H_5$ |
| Cl | H | $CF_3H$ | H | Cl | Cl | $NHCH_2CH=CH_2$ |
| Cl | H | $CF_3$ | H | F | Cl | $NHCH_2C\equiv CH$ |
| Cl | H | $CF_3$ | Cl | Cl | Br | $NHCH_2$ 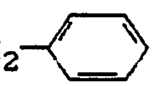 |
| Cl | H | $CF_3$ | H | H | Cl | $NHCOCH_3$ |
| Cl | H | $CF_3$ | H | Cl | Cl | $NHCOCH_3$ |
| Cl | H | $CF_3$ | H | F | Cl | $NHCOCH_3$ |
| Cl | H | $CF_3$ | F | Cl | Cl | $NHCOC_2H_5$ |
| Cl | H | $CF_3$ | H | Cl | Cl | $NHCOCHCl_2$ |
| Cl | H | $CF_3$ | H | Cl | Cl | $NHCOCF_3$ |

Tabelle 1 - Fortsetzung

| $R^1$ | $R^2$ | $R^3$ | $R^4$ | $R^5$ | X | Y |
|---|---|---|---|---|---|---|
| Cl | H | $CF_3$ | H | H | Cl | $NHCOOCH_3$ |
| Cl | H | $CF_3$ | H | Cl | Cl | $NHCOOCH_3$ |
| Cl | H | $CF_3$ | H | F | Cl | $NHCOOCH_3$ |
| Cl | H | $CF_3$ | F | F | Cl | $NHCOOC_2H_5$ |
| Cl | H | $CF_3$ | H | H | Br | $NHCOOCH_2$ 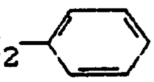 |
| Cl | H | $CF_3$ | H | H | Cl | NHCOO 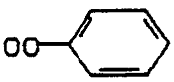 |
| Cl | H | $CF_3$ | H | H | Cl | $NHSO_2CH_3$ |
| Cl | H | $CF_3$ | H | Cl | Cl | $NHSO_2CH_3$ |
| Cl | H | $CF_3$ | H | F | Cl | $NHSO_2CH_3$ |
| Cl | H | $CF_3$ | Cl | Cl | Cl | $NHSO_2CF_3$ |
| Cl | H | $CF_3$ | F | Cl | Cl | $NHSO_2C_2H_5$ |
| Cl | H | $CF_3$ | H | F | Cl | $NHSO_2$ 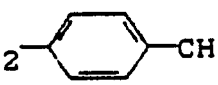 $CH_3$ |
| Cl | H | $CF_3$ | H | H | Cl | $N(COCH_3)_2$ |
| Cl | H | $CF_3$ | H | Cl | Cl | $N(COCH_3)_2$ |
| Cl | H | $CF_3$ | H | F | Cl | $N(COCH_3)_2$ |
| Cl | H | $CF_3$ | H | H | Cl | $N(SO_2CH_3)_2$ |
| Cl | H | $CF_3$ | H | Cl | Cl | $NHOCH_3$ |
| Cl | H | $CF_3$ | H | F | Cl | $NHOCH(CH_3)_2$ |

Tabelle 1 - Fortsetzung

| $R^1$ | $R^2$ | $R^3$ | $R^4$ | $R^5$ | X | Y |
|---|---|---|---|---|---|---|
| Cl | H | $CF_3$ | H | H | Cl | 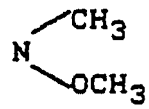 |
| Cl | H | $CF_3$ | F | Cl | Cl | $NHOCH_2CH{=}CH_2$ |
| Cl | H | $CF_3$ | Cl | Cl | Cl | $NHOCH_2CH(CH_3)_2$ |
| Cl | H | $CF_3$ | H | F | Cl | 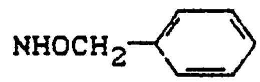 |
| Cl | H | $CF_3$ | H | Cl | Cl | 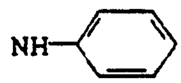 |
| Cl | H | $CF_3$ | Cl | Cl | Cl | 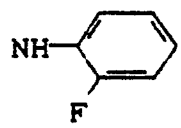 |
| Cl | H | $CF_3$ | F | F | Cl | 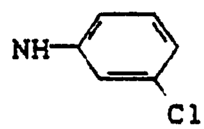 |
| Cl | H | $CF_3$ | H | F | Cl | 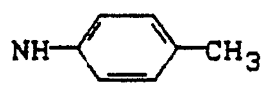 |
| Cl | H | $CF_3$ | H | Cl | Cl | 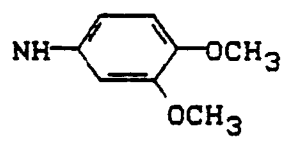 |
| Cl | H | $CF_3$ | H | H | Br | 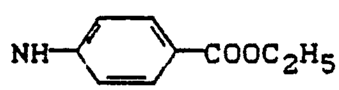 |

Tabelle 1 - Fortsetzung

| $R^1$ | $R^2$ | $R^3$ | $R^4$ | $R^5$ | X | Y |
|---|---|---|---|---|---|---|
| Cl | H | $CF_3$ | H | F | Cl | 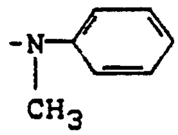 |
| Cl | H | $CF_3$ | Cl | Cl | Cl | 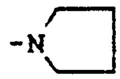 |
| Cl | H | $CF_3$ | Cl | Cl | Cl | 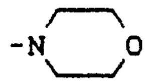 |
| Cl | H | $CF_3$ | H | F | Cl | 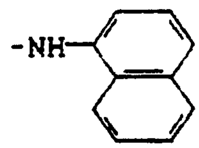 |
| Cl | H | $CF_3$ | H | F | Cl | $-N=CH-N(CH_3)_2$ |
| Cl | H | $CF_3$ | H | H | Cl | 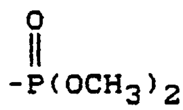 |
| Cl | H | $CF_3$ | H | Cl | Cl | 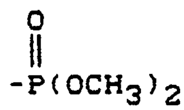 |
| Cl | H | $CF_3$ | H | F | Cl | 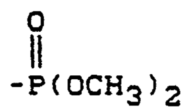 |
| Cl | H | $CF_3$ | H | H | Cl | 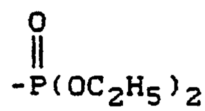 |
| Cl | H | $CF_3$ | H | Cl | Cl | 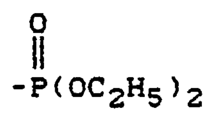 |

<u>Tabelle 1</u> - Fortsetzung

| $R^1$ | $R^2$ | $R^3$ | $R^4$ | $R^5$ | X | Y |
|---|---|---|---|---|---|---|
| Cl | H | $CF_3$ | H | F | Cl | $-\overset{O}{\overset{\|}{P}}(OC_2H_5)_2$ |
| Cl | H | $CF_3$ | H | H | Cl | -CO-Cl |
| Cl | H | $CF_3$ | H | H | Br | -CO-Cl |
| Cl | H | $CF_3$ | H | H | Cl | -COOH |
| Cl | H | $CF_3$ | H | H | Br | -COOH |
| Cl | H | $CF_3$ | H | Cl | Cl | -CO-Cl |
| Cl | H | $CF_3$ | H | F | Cl | -CO-Cl |
| Cl | H | $CF_3$ | H | H | Cl | $-CO-SCH_3$ |
| Cl | H | $CF_3$ | H | Cl | Cl | $-CO-SC_2H_5$ |
| Cl | H | $CF_3$ | H | F | Cl | $-CO-SC_3H_7$ |
| Cl | H | $CF_3$ | Cl | Cl | Cl | $-CO-SC_4H_9$ |
| Cl | H | $CF_3$ | F | Cl | Cl | $-CO-SCH_2-$ 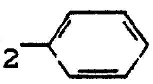 |
| Cl | H | $CF_3$ | H | Cl | Cl | $-CO-SCH_2COOCH_3$ |
| Cl | H | $CF_3$ | H | F | Cl | $-CO-SCH_2COOC_2H_5$ |
| Cl | H | $CF_3$ | H | H | Cl | $-COOCH_3$ |
| Cl | H | $CF_3$ | H | Cl | Cl | $-COOCH_3$ |
| Cl | H | $CF_3$ | Cl | Cl | Cl | $-COOCH_3$ |
| Cl | H | $CF_3$ | F | Cl | Cl | $-COOCH_3$ |
| Cl | H | $CF_3$ | H | F | Cl | $-COOCH_3$ |

<u>Tabelle 1</u> - Fortsetzung

| $R^1$ | $R^2$ | $R^3$ | $R^4$ | $R^5$ | X | Y |
|---|---|---|---|---|---|---|
| Cl | H | $CF_3$ | H | H | Cl | $-COOC_2H_5$ |
| Cl | H | $CF_3$ | H | Cl | Cl | $-COOC_2H_5$ |
| Cl | H | $CF_3$ | H | F | Cl | $-COOC_2H_5$ |
| Cl | H | $CF_3$ | Cl | Cl | Cl | $-COOC_2H_5$ |
| Cl | H | $CF_3$ | F | F | Cl | $-COOC_3H_7$ |
| Cl | H | $CF_3$ | H | F | Cl | $-COOC_4H_9$ |
| Cl | H | $CF_3$ | H | F | Cl | $-COOCH_2CH_2OCH_3$ |
| Cl | H | $CF_3$ | H | F | Cl | $-COOCH_2CH_2OC_2H_5$ |
| Cl | H | $CF_3$ | H | F | Cl | $-COOCH_2CH_2SCH_3$ |
| Cl | H | $CF_3$ | H | F | Cl | $-COOCH_2CH_2SC_2H_5$ |
| Cl | H | $CF_3$ | H | Cl | Cl | $-COOCH_2CH_2SO_2CH_3$ |
| Cl | H | $CF_3$ | H | H | Cl | $-COOCH_2CH_2OCH_2-$ 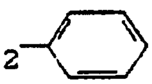 |
| Cl | H | $CF_3$ | H | F | Cl | $-COOCH_2CH_2SCH_2-$ 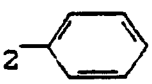 |
| Cl | H | $CF_3$ | H | Cl | Cl | $-COOCH_2COOCH_3$ |
| Cl | H | $CF_3$ | Cl | Cl | Cl | $-COOCH_2COOC_2H_5$ |
| Cl | H | $CF_3$ | H | F | Cl | $-COOCH-COOCH_3$<br>\|<br>$CH_3$ |
| Cl | H | $CF_3$ | H | F | Cl | $-COOCHCOOC_2H_5$<br>\|<br>$CH_3$ |

Tabelle 1 - Fortsetzung

| $R^1$ | $R^2$ | $R^3$ | $R^4$ | $R^5$ | X | Y |
|---|---|---|---|---|---|---|
| Cl | H | $CF_3$ | Cl | Cl | Cl | 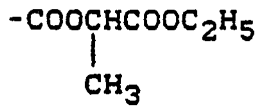 |
| Cl | H | $CF_3$ | H | Cl | Cl | 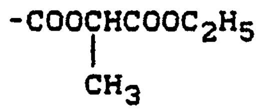 |
| Cl | H | $CF_3$ | H | Cl | Cl | $-COOCH_2-COOC_2H_5$ |
| Cl | H | $CF_3$ | H | F | Cl | $-COOCH_2COOC_2H_5$ |
| Cl | H | $CF_3$ | H | Cl | Cl | 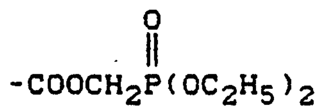 |
| Cl | H | $CF_3$ | H | Cl | Cl | $-CO-NH_2$ |
| Cl | H | $CF_3$ | H | F | Cl | $-CO-NH_2$ |
| Cl | H | $CF_3$ | H | H | Cl | $-CO-NH_2$ |
| Cl | H | $CF_3$ | Cl | Cl | Cl | $-CO-NH_2$ |
| Cl | H | $CF_3$ | H | Cl | Cl | $-CO-NHCH_3$ |
| Cl | H | $CF_3$ | H | F | Cl | $-CO-NHC_2H_5$ |
| Cl | H | $CF_3$ | H | F | Cl | $-CO-NHCH_2COOC_2H_5$ |
| Cl | H | $CF_3$ | H | F | Cl | 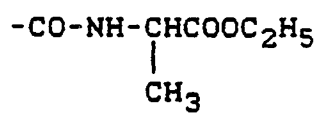 |
| Cl | H | $CF_3$ | H | H | Cl | 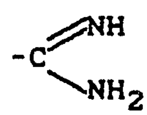 |
| Cl | H | $CF_3$ | H | F | Cl | 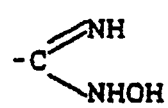 |

<u>Tabelle 1</u> - Fortsetzung

| $R^1$ | $R^2$ | $R^3$ | $R^4$ | $R^5$ | X | Y |
|---|---|---|---|---|---|---|
| Cl | H | $CF_3$ | H | Cl | Cl | 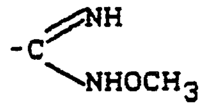 |
| Cl | H | $CF_3$ | H | F | Cl | 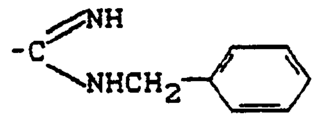 |
| Cl | H | $CF_3$ | Cl | Cl | Cl | 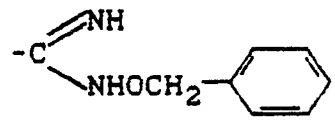 |
| Cl | H | $CF_3$ | H | H | Cl | $-CO-NH-SO_2CH_3$ |
| Cl | H | $CF_3$ | H | Cl | Cl | $-CO-NH-SO_2CH_3$ |
| Cl | H | $CF_3$ | H | F | Cl | $-CO-NH-SO_2CH_3$ |
| Cl | H | $CF_3$ | H | F | Cl | $-CO-NH-SO_2C_2H_5$ |
| Cl | H | $CF_3$ | F | Cl | Cl | $-CO-NH-SO_2CH_3$ |
| Cl | H | $CF_3$ | Cl | Cl | Cl | $-CO-NH-SO_2CH_3$ |
| Cl | H | $CF_3$ | F | F | Cl | $-CO-NH-SO_2C_2H_5$ |
| Cl | H | $CF_3$ | H | Cl | Cl | $-CO-NH-SO_2C_2H_5$ |
| Cl | H | $CF_3$ | H | Cl | Cl | $-CO-NH-SO_2$ 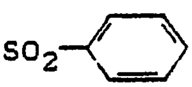 |
| Cl | H | $CF_3$ | H | F | Cl | $-CO-NHCH_2$ 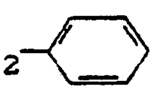 |
| Cl | H | $CF_3$ | H | F | Cl | -CO-NH 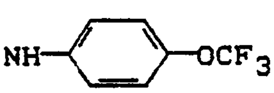 |

**Tabelle 1** - Fortsetzung

| $R^1$ | $R^2$ | $R^3$ | $R^4$ | $R^5$ | X | Y |
|---|---|---|---|---|---|---|
| Cl | H | $CF_3$ | H | Cl | Cl | $-COOCH_2$ 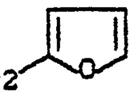 |
| Cl | H | $CF_3$ | H | F | Cl | $-COOCH_2$ 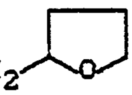 |
| Cl | H | $CF_3$ | Cl | Cl | Cl | $-COOCH_2CH_2$ 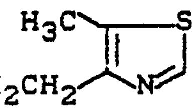 |
| Cl | H | $CF_3$ | H | F | Cl | $-COOCH_2$ 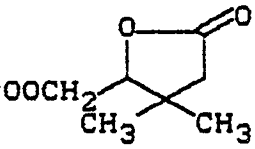 |
| Cl | H | $CF_3$ | H | Cl | Cl | $-COOCH_2$ 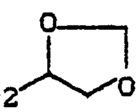 |
| Cl | H | $CF_3$ | H | F | Cl | $-COOCH_2$ 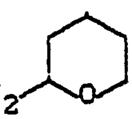 |
| Cl | H | $CF_3$ | H | F | Cl | -COO 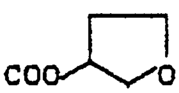 |
| Cl | H | $CF_3$ | H | Cl | Cl | $-COOCH_2$ 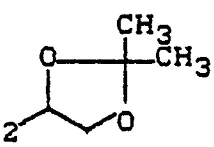 |
| Cl | H | $CF_3$ | H | Cl | Cl | $-COOCH_2CH_2$ 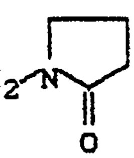 |
| Cl | H | $CF_3$ | H | Cl | Cl | -COOH |

<u>Tabelle 1</u> - Fortsetzung

| $R^1$ | $R^2$ | $R^3$ | $R^4$ | $R^5$ | X | Y |
|---|---|---|---|---|---|---|
| Cl | H | $CF_3$ | H | F | Cl | -COOH |
| Cl | H | $CF_3$ | Cl | Cl | Cl | -COOH |
| Cl | H | $CF_3$ | F | Cl | Cl | -COOH |
| Cl | H | $CF_3$ | H | F | Br | -COOH |
| Cl | H | $CF_3$ | Cl | Cl | Cl | -CO-Cl |
| Cl | H | $CF_3$ | F | Cl | Cl | -CO-Cl |

Verwendet man für das erfindungsgemäße Verfahren (a) 2-Chlor-5-(2,6-difluor-4-trifluormethyl-phenoxy)-toluol und Brom als Ausgangsstoffe, so kann der Reaktionsablauf durch das folgende Formelschema wiedergegeben werden:

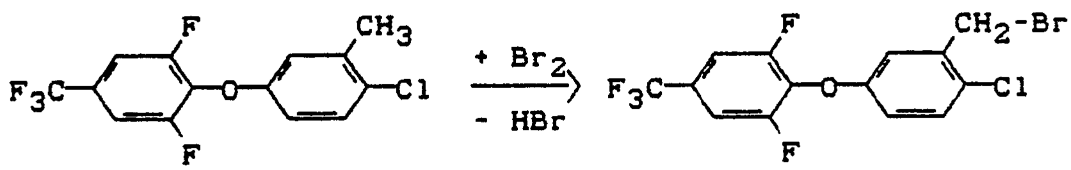


Verwendet man für das erfindungsgemäße Verfahren (b) 2-Brom-5-(2-chlor-6-fluor-4-trifluormethylphenoxy)-benzylbromid und Kaliumcyanid als Ausgangsstoffe, so kann der Reaktionsablauf durch das folgende Formelschema wiedergegeben werden:

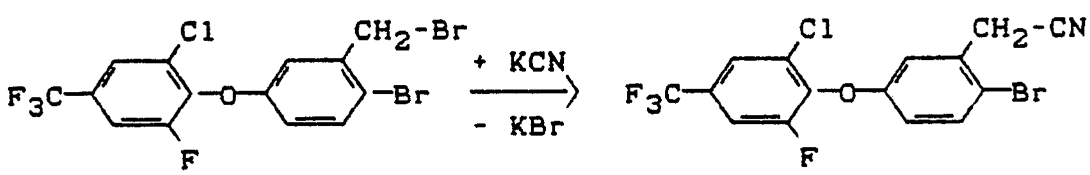


Verwendet man für das erfindungsgemäße Verfahren (c) 2-Chlor-5-(2,3,6-trifluor-4-trifluormethyl-phenoxy)-benzylchlorid und Trimethylphosphit als Ausgangsstoffe, so kann der Reaktionsablauf durch das folgende Formelschema wiedergegeben werden:

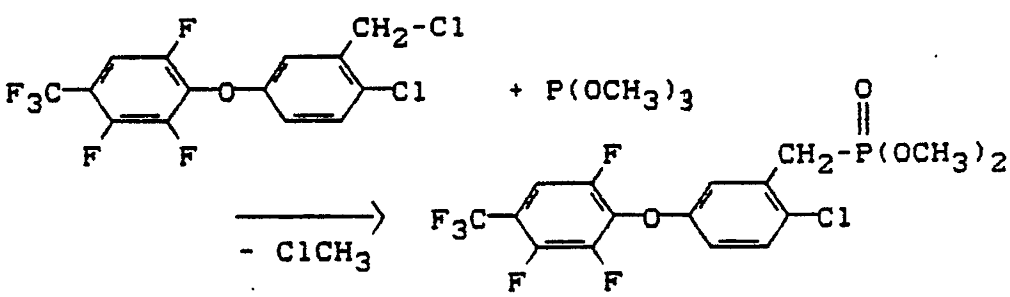


Verwendet man für das erfindungsgemäße Verfahren (d) 2-Chlor-5-(2,3,6-trichlor-4-trifluormethyl-phenoxy)-benzylcyanid und O-Methyl-hydroxylamin als Ausgangsstoffe, so kann der Reaktionsablauf durch das

folgende Formelschema wiedergegeben werden:

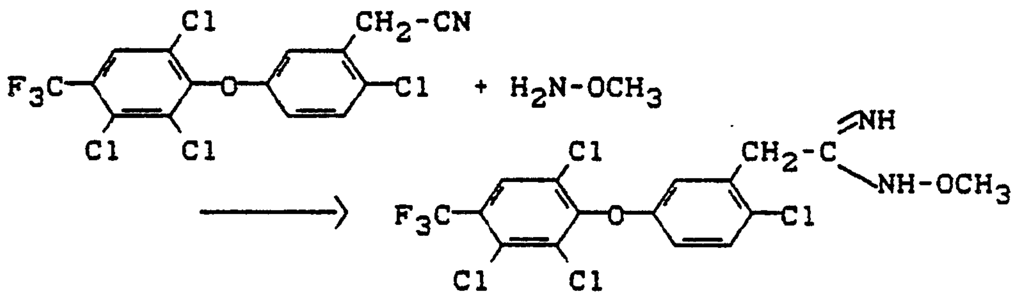

Verwendet man für das erfindungsgemäße Verfahren (e) 2-Brom-5-(2,6-dichlor-3-fluor-4-trifluormethyl-phenoxy)-phenylessigsäure-methylester und Wasser als Ausgangsstoffe, so kann der Reaktionsablauf durch das folgende Formelschema wiedergegeben werden:

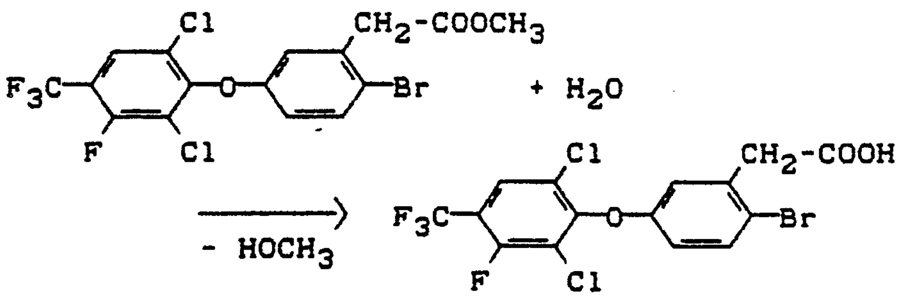


Verwendet man für das erfindungsgemäße Verfahren (f) 2-Brom-5-(2-chlor-4-trifluormethyl-phenoxy)-phenylessigsäure und Thionylchlorid als Ausgangsstoffe, so kann der Reaktionsablauf durch das folgende Formelschema wiedergegeben werden:

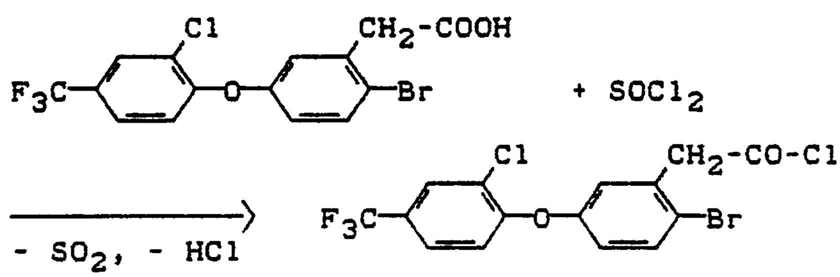


Verwendet man für das erfindungsgemäße Verfahren (g) 2-Chlor-5-(2,3,6-trichlor-4-trifluormethyl-phenoxy)-phenylessigsäure-chlorid und Mercaptoessigsäuremethylester als Ausgangsstoffe, so kann der Reaktionsablauf durch das folgende Formelschema wiedergegeben werden:

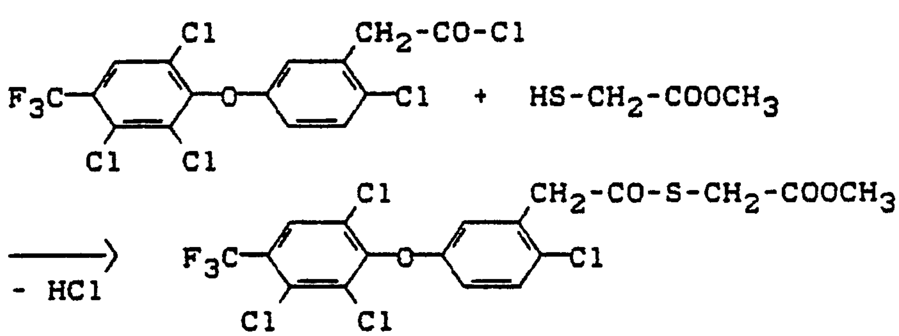

Verwendet man für das erfindungsgemäße Verfahren (h) 2-Chlor-5-(2,6-dichlor-4-trifluormethyl-phenoxy)-benzylchlorid und Butyllithium, dann Aceton und schließlich Kohlensäure als Ausgangsstoffe, so kann der Reaktionsablauf durch das folgende Formelschema wiedergegeben werden:

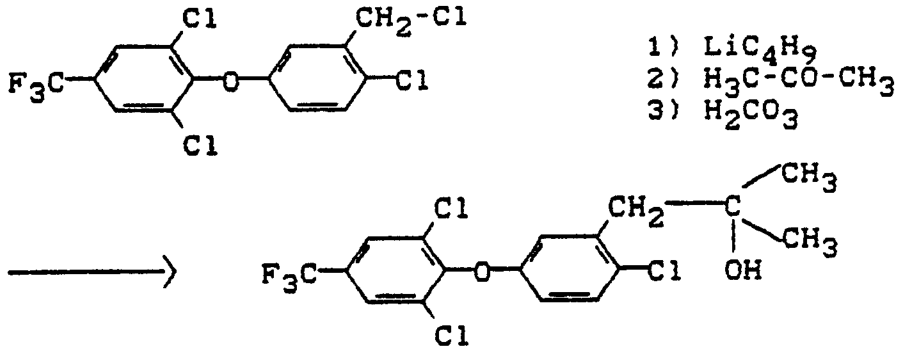


Verwendet man für das erfindungsgemäße Verfahren (i) 3-(2,3,6-Trichlor-4-trifluormethyl-phenoxy)-benzylbromid und Chlor als Ausgangsstoffe, so kann der Reaktionsablauf durch das folgende Formelschema wiedergegeben werden:

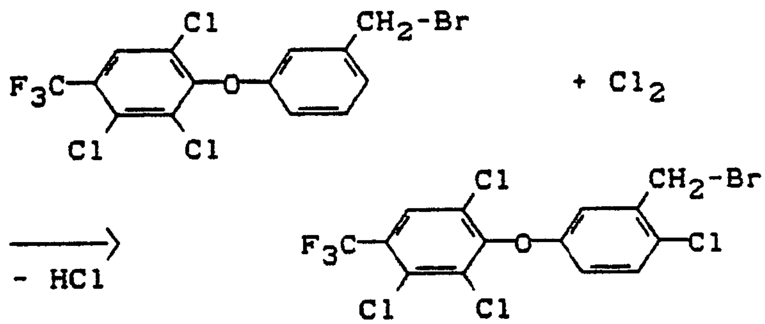


Die beim erfindungsgemäßen Verfahren (a) zur Herstellung von Verbindungen der Formel (I) als Ausgangsstoffe zu verwendenden halogensubstituierten Phenoxytoluol-Derivate sind durch die Formel (II) allgemein definiert.

In Formel (II) haben $R^1$, $R^2$, $R^3$, $R^4$, $R^5$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$, $R^5$ und X angegeben wurden.

Beispiele für die Ausgangsstoffe der Formel (II) sind in der nachstehenden Tabelle 2 aufgeführt.

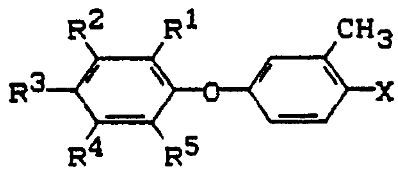

(II)

Tabelle 2: Beispiele für die Ausgangsstoffe der Formel (II)

| $R^1$ | $R^2$ | $R^3$ | $R^4$ | $R^5$ | X |
|---|---|---|---|---|---|
| Cl | H | $CF_3$ | H | H | Cl |
| Cl | H | $CF_3$ | H | H | Br |
| Cl | H | $CF_3$ | H | H | F |
| Cl | H | $CF_3$ | H | Cl | Cl |
| Cl | H | $CF_3$ | H | Cl | Br |
| Cl | H | $CF_3$ | H | Cl | F |
| Cl | H | $CF_3$ | H | F | Cl |
| Cl | H | $CF_3$ | H | F | Br |
| Cl | H | $CF_3$ | H | F | F |
| Cl | H | $CF_3$ | Cl | Cl | Cl |
| Cl | H | $CF_3$ | Cl | Cl | Br |
| Cl | H | $CF_3$ | Cl | Cl | F |

Tabelle 2 - Fortsetzung

| $R^1$ | $R^2$ | $R^3$ | $R^4$ | $R^5$ | X |
|---|---|---|---|---|---|
| Cl | H | $CF_3$ | F | Cl | Cl |
| Cl | H | $CF_3$ | F | Cl | Br |
| Cl | H | $CF_3$ | F | Cl | F |
| Cl | H | $CF_3$ | F | F | Cl |
| Cl | H | $CF_3$ | F | F | Br |
| Cl | H | $CF_3$ | F | F | F |
| Cl | F | $CF_3$ | F | Cl | Cl |
| Cl | F | $CF_3$ | F | Cl | Br |
| Cl | F | $CF_3$ | F | Cl | F |
| Cl | F | $CF_3$ | F | F | Cl |
| Cl | F | $CF_3$ | F | F | Br |
| Cl | F | $CF_3$ | F | F | F |

Die Ausgangsstoffe der Formel (II) sind bekannt und/oder können nach an sich bekannten Verfahren hergestellt werden (vgl. DE-AS 23 04 006).

Verfahren (a) wird unter Einsatz eines Halogenierungsmittels durchgeführt. Bevorzugte Halogenierungsmittel sind solche, die zur Einführung von Halogen-Substituenten in Seitenketten von Aromaten geeignet sind. Als Beispiele für solche Halogenierungsmittel seien elementares Chlor oder Brom, N-Chlor- oder N-Brom-succinimid und Sulfurylchlorid genannt.

Verfahren (a) wird gegebenenfalls unter Belichtung durchgeführt. Bevorzugte Lichtquellen sind solche, welche, wie z. B. Quecksilbertauchlampen, energiereicheres Licht als das übliche Tageslicht liefern.

Verfahren (a) wird gegebenenfalls in Gegenwart von Katalysatoren durchgeführt. Bevorzugte Katalysatoren sind Stoffe, die zur Radikalerzeugung geeignet sind. Als Beispiele für geeignete Katalysatoren seien Benzoylperoxid und Azo-bis-isobutyronitril genannt.

Verfahren (a) wird gegebenenfalls in Gegenwart eines Verdünnungsmittels durchgeführt. Als solche kommen vorzugsweise die gegenüber den üblichen Halogenierungsmitteln relativ inerten organischen Lösungsmittel in Betracht. Hierzu gehören insbesondere halogenierte Kohlenwasserstoffe, wie Choroform, Tetrachlormethan, Chlorbenzol und o-Dichlorbenzol.

Die Reaktionstemperaturen können bei dem erfindungsgemäßen Verfahren (a) in einem größeren Bereich variiert werden. Im allgemeinen arbeitet man bei Temperaturen zwischen 0 °C und 200 °C, vorzugsweise bei Temperaturen zwischen 20 °C und 150 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahren (a) setzt man je Mol Ausgangsverbindung der Formel (II) im allgemeinen zwischen 0,8 und 2,0 Mol, vorzugsweise zwischen 1,0 und 1,5 Mol, Halogenierungsmittel ein. Die Reaktionskomponenten werden im allgemeinen bei Raumtemperatur vermischt und das Reaktionsgemisch wird - gegebenenfalls bei erhöhter Temperatur - bis zum Ende der Umsetzung gerührt. Die Produkte der Formel (I) können dann nach üblichen Methoden, beispielsweise durch Säulenchromatographie, rein erhalten werden.

Die beim erfindungsgemäßen Verfahren (b) zur Herstellung von Verbindungen der Formel (I) als

Ausgangsstoffe zu verwendenden halogensubstituierten Phenoxybenzylhalogenide sind durch die Formel (Ia) allgemein definiert.

In Formel (Ia) haben $R^1$, $R^2$, $R^3$, $R^4$, $R^5$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$ $R^3$, $R^4$, $R^5$ und X angegeben wurden und $X^1$ steht vorzugsweise für Chlor oder Brom.

Beispiele für die Ausgangsstoffe der Formel (Ia) sind Tabelle 1 (Y = $X^1$ : Cl, Br) zu entnehmen.

Die Ausgangsstoffe der Formel (Ia) sind erfindungsgemäße Verbindungen und mit Ausnahme der durch Disclaimer ausgenommenen Verbindungen noch nicht aus der Literatur bekannt. Sie können nach dem erfindungsgemäßen Verfahren (a) hergestellt werden.

Die beim erfindungsgemäßen Verfahren (b) weiter als Ausgangsstoffe zu verwendenden nucleophilen Verbindungen sind durch die Formel (III) allgemein definiert.

In Formel (III) hat Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Y angegeben wurden und M steht vorzugsweise für Wasserstoff, Natrium oder Kalium.

Als Beispiele für die Ausgangsstoffe der Formel (III) seien genannt:
Natrium- oder Kalium-iodid, Natrium- oder Kaliumcyanid, Natrium- oder Kalium-thiocyanat, Natrium- oder Kalium-cyanamid, Malonsäure-dimethylester und -diethylester sowie deren Natrium- und Kaliumsalze, Acetessigester, Ammoniak, Methylamin, Ethylamin, Propylamin, Isopropylamin, Butylamin, Isobutylamin, sec-Butylamin, tert-Bu tylamin, Dimethylamin, Diethylamin, Dipropylamin, Diisopropylamin, Dibutylamin, Diisobutylamin, Allylamin, Diallylamin, Propargylamin, Dipropargylamin, Benzylamin, Dibenzylamin, Hydroxylamin, O-Methyl-, O-Ethyl-, O-Propyl-, O-Isopropyl-, O-Butyl-, O-Isobutyl- und O-sec-Butyl-hydroxylamin, O-Allyl-hydroxylamin, O-Benzyl-hydroxylamin, N,O-Dimethyl-hydroxylamin, Anilin, o-, m-und p-Toluidin, N-Methyl-anilin, N-Ethyl-anilin, N-Propyl-anilin, N-Isopropyl-anilin, Pyrrolidin, Piperidin und Morpholin.

Die Ausgangsstoffe der Formel (III) sind bekannte organische Synthesechemikalien.

Das erfindungsgemäße Verfahren (b) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethyl sulfoxid, Tetramethylensulfon und Hexamethylphosphorsäuretriamid.

Die genannten organischen Lösungsmittel können bei Verfahren (b) gegebenenfalls auch in Kombination mit Wasser verwendet werden.

Als Säureakzeptoren können bei dem erfindungsgemäßen Verfahren (b) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 10 °C und 100 °C.

Verfahren (b) wird im allgemeinen bei Normaldruck durchgeführt.

Zur Durchführung des erfindungsgemäßen Verfahrens (b) werden die jeweils benötigten Ausgangsstoffe im allge meinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden.

Beispielsweise wird das Reaktionsgemisch - gegebenenfalls nach Einengen - mit Wasser verdünnt und das gewünschte Reaktionsprodukt wird mit einem mit Wasser praktisch nicht mischbaren organischen

Lösungsmittel, z. B. Methylenchlorid, Chloroform, Diethylether, Toluol oder Xylol, extrahiert, Die organische Extraktionslösung wird mit Wasser gewaschen, mit einem üblichen Trockenmittel, wie z. B. Natriumsulfat, getrocknet und filtriert. Nach dem Einengen des Filtrate erhält man die Verbindungen der Formel (I) als Rohprodukte, welche auf übliche Weise, z. B. chromatographisch und/oder durch Umkristallisation gereinigt werden können.

Die beim erfindungsgemäßen Verfahren (c) zur Herstellung von Verbindungen der Formel (I) als Ausgangsstoffe zu verwendenden halogensubstituierten Phenoxybenzylhalogenide der Formel (Ia) wurden bereits bei der Beschreibung des Verfahrens (b) näher beschrieben.

Die beim erfindungsgemäßen Verfahren (c) weiter als Ausgangsstoffe zu verwendenden Alkoxy-Phosphorverbindungen sind durch die Formel (IV) allgemein definiert.

In Formel (IV) haben $R^{14}$ und $R^{15}$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^{14}$ und $R^{15}$ angegeben wurden und R steht vorzugsweise für $C_1$-$C_4$-Alkyl, insbesondere für $C_1$-$C_3$-Alkyl.

Als Beispiele für die Ausgangsstoffe der Formel (IV) seien genannt:
Trimethyl-, Triethyl-, Tripropyl-, Triisopropyl-, Tributyl- und Tris-(2-chlor-ethyl)-phosphit.

Die Ausgangsstoffe der Formel (IV) sind bekannte organische Synthesechemikalien.

Das erfindungsgemäße Verfahren (c) wird gegebenenfalls in Gegenwart von Verdünnungsmittteln durchgeführt. Es kommen vor allem diejenigen organischen Lösungsmittel in Betracht, die bereits oben für Verfahren (b) als Verdünnungsmittel angegeben wurden. Vorzugsweise wird Verfahren (c) jedoch ohne gesondertes Verdünnungsmittel, im allgemeinen aber unter Verwendung überschüssiger Alkoxy-Phosphorverbindung der Formel (IV) als Verdünnungsmittel, durchgeführt.

Die Reaktionstemperaturen können bei dem erfindungsgemäßen Verfahren (c) in einem größeren Bereich variiert werden. Im allgemeinen arbeitet man bei Temperaturen zwischen 0 °C und 200 °C, vorzugsweise bei Temperaturen zwischen 20 °C und 150 °C.

Verfahren (c) wird im allgemeinen bei Normaldruck durchgeführt.

Zur Durchführung des erfindungsgemäßen Verfahrens (c) setzt man je Mol Ausgangsverbindung der Formel (Ia) im allgemeinen zwischen 1 und 100 Mol, vorzugsweise zwischen 2 und 50 Mol, Alkoxy-Phosphorverbindung der Formel (IV) ein.

Die Reaktionskomponenten werden im allgemeinen bei Raumtemperatur vermischt und das Reaktionsgemisch wird - vorzugsweise bei erhöhter Temperatur - bis zum Ende der Umsetzung gerührt. Die Produkte der Formel (I) können dann nach üblichen Methoden, beispielsweise wie oben für die erfindungsgemäßen Verfahren (a) und (b) angegeben, rein erhalten werden.

Die beim erfindungsgemäßen Verfahren (d) zur Herstellung von Verbindungen der Formel (I) als Ausgangsstoffe zu verwendenden halogensubstituierten Phenoxybenzylcyanide sind durch die Formel (Ib) allgemein definiert.

In Formel (Ib) haben $R^1$, $R^2$, $R^3$, $R^4$, $R^5$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$, $R^5$ und X angegeben wurden.

Beispiele für die Ausgangsstoffe der Formel (Ib) sind Tabelle 1 (Y = CN) zu entnehmen.

Die Ausgangsstoffe der Formel (Ib) sind erfindungsgemäße Verbindungen und mit Ausnahme der durch Disclaimer ausgenommenen Verbindung - 2-Chlor-5-(2-chlor-4-trifluormethyl-phenoxy)-benzylcyanid - noch nicht aus der Literatur bekannt. Sie können nach dem erfindungsgemäßen Verfahren (b) hergestellt werden.
Die beim erfindungsgemäßen Verfahren (d) zur Herstellung von Verbindungen der Formel (I) als Ausgangsstoffe zu verwendenden nucleophilen Verbindungen sind durch die Formel (V) allgemein definiert.

In Formel (V) hat Z vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für Z angegeben wurden und $M^1$ steht vorzugsweise für Wasserstoff, Natrium oder Kalium.

Als Beispiele für die Ausgangsstoffe der Formel (V) seien genannt:
Ammoniak, Methylamin, Ethylamin, Propylamin, Isopropylamin, Butylamin, Isobutylamin, sec-Butylamin, tert-Butylamin, Dimethylamin, Diethylamin, Dipropylamin, Allylamin, Propargylamin, Benzylamin, Hydroxylamin, O-Methyl-, O-Ethyl-, O-Propyl-, O-Isopropyl-, O-Butyl-, O-Isobutyl-, O-Allyl- und O-Benzyl-hydroxylamin, Methanol, Ethanol, Propanol und Butanol.

Die Ausgangsstoffe der Formel (V) sind bekannte organische Synthesechemikalien.

Das erfindungsgemäße Verfahren (d) wird gegebenenfalls in Gegenwart eines Verdünnungsmittels duchgeführt. Als Verdünnungsmittel kommen hierbei vorzugsweise polare organische Solventien, insbesondere Alkohole, wie Methanol, Ethanol, Propanol, Isopropanol, Butanol, Isobutanol, sec-Butanol und tert-Butanol in Betracht.

Verfarhen (d) wird gegebenenfalls in Gegenwart eines Katalysators durchgeführt. Als solche kommen vorzugsweise starke Säuren, wie z.B. Chlorwasserstoffsäure, Bromwasserstoffsäure, Schwefelsäure, Methansulfonsäure, Benzolsulfonsäure und p-Toluolsulfonsäure, in Betracht.

Die Reaktionstemperaturen können bei dem erfindungsgemäßen Verfahren (d) in einem größeren Bereich variiert werden. Im allgemeinen arbeitet man bei Temperaturen zwischen -20 °C und +80 °C, vorzugsweise bei Temperaturen zwischen 0 °C und 50 °C.

Verfahren (d) wird im allgemeinen bei Normaldruck durchgeführt.

Zur Durchführung des erfindungsgemäßen Verfahrens (d) setzt man je Mol Ausgangsverbindung der Formel (Ib) im allgemeinen zwischen 1 und 500 Mol, vorzugsweise zwischen 2 und 200 Mol, nucleophile Verbindung der Formel (V) ein. Die Reaktionskomponenten werden im allgemeinen unter Kühlen vermischt und das Reaktionsgemisch wird dann bis zum Ende der Umsetzung gerührt. Die Produkte der Formel (I) können dann nach üblichen Methoden, beispielsweise wie oben für die erfindungsgemäßen Verfahren (a) und (b) angegeben, rein erhalten werden.

Die beim erfindungsgemäßen Verfahren (e) zur Herstellung von Verbindungen der Formel (I) als Ausgangsstoffe zu verwendenden halogensubstituierten Phenoxybenzylverbindungen sind durch die Formel (Ic) allgemein definiert.

In Formel (Ic) haben $R^1$, $R^2$, $R^3$, $R^4$, $R^5$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$, $R^5$ und X angegeben wurden und $Y^1$ steht vorzugsweis für Cyano, Methoxycarbonyl oder Ethoxycarbonyl.

Beispiele für die Ausgangsstoffe der Formel (Ic) sind Tabelle 1 ($Y = Y^1$ : CN, $COOCH_3$, $COOC_2H_5$) zu entnehmen.

Die Ausgangsstoffe der Formel (Ic) sind erfindungsgemäße Verbindungen und mit Ausnahme der durch Disclaimer ausgenommenen Verbindung - 2-Chlor-5-(2-chlor-4-trifluormethyl-phenoxy)-benzylcyanid - noch nicht aus der Literatur bekannt. Sie können nach den erfindungsgemäßen Verfahren (b) bzw. (d) hergestellt werden.

Verfahren (e) wird vorzugsweise in Gegenwart eines Verseifungshilfsmittels durchgeführt. Als solche kommen insbesondere starke Säuren, wie z. B. Salzsäure oder Schwefelsäure, oder Alkalihydroxide, wie z. B. Natriumhydroxid oder Kaliumhydroxid, in Betracht.

Verfahren (e) wird in Gegenwart von Wasser und gegebenenfalls in Gegenwart eines organischen Lösungsmittels durchgeführt. Als organische Lösungsmittel werden vorzugsweise Alkohole, wie z. B. Methanol oder Ethanol, eingesetzt.

Die Reaktionstemperaturen können bei dem erfindungsgemäßen Verfahren (e) in einem größeren Bereich variiert werden. Im allgemeinen arbeitet man bei Temperaturen zwischen 10 °C und 150 °C, vorzugsweise bei Temperaturen zwischen 20 °C und 100 °C.

Das erfindungsgemäße Verfahren (e) wird im allgemeinen unter Normaldruck durchgeführt. Es ist jedoch auch möglich, unter erhöhtem oder vermindertem Druck zu arbeiten.

Zur Durchführung von Verfahren (e) werden je Mol Ausgangsverbindung der Formel (Ic) im allgemeinen zwischen 0,1 und 10 Mol, vorzugsweise zwischen 0,5 und 2 Mol, Verseifungshilfsmittel eingesetzt. Die Reaktionskomponenten werden im allgemeinen bei Raumtempertur zusammen gegeben und das Reaktionsgemisch wird, gegebenenfalls bei erhöhter Temperatur, bis zum Reaktionsende gerührt. Das gegebenenfalls nach Einengen, Abkühlen und Ansäuern kristallin anfallende Reaktionsprodukt kann durch Absaugen isoliert werden.

Die beim erfindungsgemäßen Verfahren (f) zur Herstellung von Verbindungen der Formel (I) als Ausgangsstoffe zu verwendenden halogensubstituierten Phenoxyphenylessigsäuren sind durch die Formel (Id) allgemein definiert.

In Formel (Id) haben $R^1$, $R^2$, $R^3$, $R^4$, $R^5$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$, $R^5$ und X angegeben wurden.

Beispiele für die Ausgangsstoffe der Formel (Id) sind Tabelle 1 (Y = COOH) zu entnehmen.

Die Ausgangsstoffe der Formel (Id) sind erfindungsgemäße Verbindungen und können nach dem erfindungsgemäßen Verfahren (e) hergestellt werden.

Verfahren (f) wird unter Verwendung eines Säurehalogenids durchgeführt. Es können die üblichen Mittel für die Umsetzung von Carbonsäuren zu Carbonsäurehalogeniden eingesetzt werden. Als Beispiele hierfür seien Phosgen, Thionylchlorid und Phosphorylchlorid genannt. Vorzugsweise wird Thionylchlorid verwendet.

Verfahren (f) wird gegebenenfalls in Gegenwart eines Katalysators durchgeführt. Es können die für die Herstel lung von Säurehalogeniden aus Säuren üblichen Katalysatoren, wie z. B. Pyridin oder Dimethylformamid, verwendet werden.

Verfahren (f) wird gegebenenfalls in Gegenwart eines Verdünnungsmittels durchgeführt. Vorzugsweise kommen inerte organische Lösungsmittel aus der Reihe der halogenierten Kohlenwasserstoffe, wie z. B. Methylenchlorid, Chloroform, Tetrachlormethan oder 1,2-Dichlorethan, in Betracht.

Die Reaktionstemperaturen können bei dem erfindungsgemäßen Verfahren (f) in einem größeren Bereich variiert werden. Im allgemeinen arbeitet man bei Temperaturen zwischen 0 °C und 100 °C, vorzugsweise bei Temperaturen zwischen 10 °C und 90 °C.

Verfahren (f) wird im allgemeinen bei Normaldruck durchgeführt.

Zur Durchführung von Verfahren (f) werden je Mol Ausgangsverbindung der Formel (Id) im allgemeinen zwischen 1 und 100 Mol, vorzugsweise zwischen 2 und 50 Mol, Säurehalogenid eingesetzt. Die Reaktionskomponenten werden im allgemeinen bei Raumtemperatur zusammengegeben und das Reaktionsgemisch wird, gegebenenfalls bei erhöhter Temperatur bis zum Ende der Umsetzung gerührt. Das nach Abdestillieren der flüchtigen Komponenten unter vermindertem Druck verbleibende Reaktionsprodukt kann durch Umkristallisation gereinigt werden, aber auch ohne weitere Reinigung für Folgeumsetzungen eingesetzt werden.

Die beim erfindungsgemäßen Verfahren (g) zur Herstellung von Verbindungen der Formel (I) als Ausgangsstoffe zu verwendenden halogensubstituierten Phenoxyphenylessigsäurehalogenide sind durch die Formel (Ie) allgemein definiert.

In Formel (Ie) haben $R^1$, $R^2$, $R^3$, $R^4$, $R^5$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$, $R^5$ und X angegeben wurden.

Beispiele für die Ausgangsstoffe der Formel (Ie) sind Tabelle 1 (Y = CO-$X^2$ : CO-Cl) zu entnehmen.

Die Ausgangsstoffe der Formel (e) sind erfindungsgemäße Verbindungen und können nach dem erfindungsgemäßen Verfahren (f) hergestellt werden.

Die beim erfindungsgemäßen Verfahren (g) weiter als Ausgangsstoffe zu verwendenden nucleophilen Verbindungen sind durch die Formel (V) allgemein definiert.

In Formel (V) hat Z vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für Z angegeben wurde und $M^1$ steht vorzugsweise für Wasserstoff, Natrium oder Kalium.

Als Beispiele für die Ausgangsstoffe der Formel (V) seien genannt:

Methylamin, Ethylamin, Propylamin, Isopropylamin, Cyanamid, Dimethylamin, Diethylamin, Hyroxylamin, O-Methylhydroxylamin, Hydrazin, Methylsulfonylhydrazin, Methanol, Ethanol, Propanol, Isopropanol, Butanol, Isobutanol, sec-Butanol, 2-Methoxy-ethanol, 2-Ethoxy-ethanol, 2-Methylthio-ethanol, 2-Ethylthio-ethanol, 2-Benzyloxy-ethanol, 2-Benzylthio-ethanol, Hydroxymethanphosphonsäure-diethylester und -dimethylester, 1-Hydroxy-ethanphosphonsäure-dimethylester und -diethylester, 1-Hydroxy-1-phenyl-methanphosphonsäure-dimethylester und -diethylester, 3-Hydroxyfuran, Furfurylalkohol, Perhydrofurfurylalkohol, Milchsäure-methylester und -ethylester und Glycolsäure-methylester und -ethylester.

Diese Verbindungen sind bekannte Synthesechemikalien.

Das erfindungsgemäße Verfahren (g) wird vorzugsweise in Gegenwart von Verdünnungsmitteln durchgeführt. Als solche kommen vor allem diejenigen organischen Lösungsmittel in Betracht, die bereits oben für Verfahren (b) als Verdünnungsmittel angegeben wurden.

Das erfindungsgemäße Verfahren (g) wird vorzugsweise in Gegenwart von Säureakzeptoren durchgeführt. Als solche kommen vor allem diejenigen Säurebindemittel in Betracht, die bereits oben für Verfahren (b) als Säureakzeptoren angegeben wurden.

Die Reaktionstemperaturen können bei dem erfindungsgemäßen Verfahren (g) in einem größeren Bereich variiert werden. Im allgemeinen arbeitet man bei Temperaturen zwischen 0 °C und 100 °C, vorzugsweise bei Temperaturen zwischen 10 °C und 50 °C.

Das erfindungsgemäße Verfahren (g) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (g) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Umsetzungen werden im allgemeinen in einem geeigneten Verdünnungsmittel in Gegenwart eines Säureakzeptors durchgeführt und das Reaktionsgemisch wird mehrere Stunden bei der jeweils erforderlichen Temperatur gerührt.

Die Aufarbeitung erfolgt bei dem erfindungsgemäßen Verfahren (g) jeweils nach üblichen Methoden. Beispielsweise wird das Reaktionsgemisch - gegebenenfalls nach Einengen - mit Wasser verdünnt und das gewünschte Reaktionsprodukt wird mit einem mit Wasser praktisch nicht mischbaren organischen Lösungsmittel, z. B. Methylenchlorid, Chloroform, Diethylether, Toluol oder Xylol, extrahiert. Die organische Extraktionslösung wird mit Wasser gewaschen, mit einem üblichen Trockenmittel, wie z. B. Natriumsulfat, getrocknet und filtriert. Nach dem Einengen des Filtrats erhält man die Verbindungen der Formel (I) als Rohprodukte, welche auf übliche Weise, z. B. chromatographisch und/oder durch Umkristallisation gereinigt werden können.

Die beim erfindungsgemäßen Verfahren (h) zur Herstellung von Verbindungen der Formel (I) als Ausgangsstoffe zu verwendenden halogensubstituierten Phenoxybenzylhalogenide der Formel (Ia) wurden bereits bei Verfahren (b) näher beschrieben.

Die beim erfindungsgemäßen Verfahren (h) weiter als als Ausgangsstoffe zu verwendenden Carbonylverbindungen sind durch die Formel (VI) allgemein definiert.

In Formel (VI) haben $R^8$ und $R^9$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^8$ und $R^9$ angegeben wurden.

Als Beispiele für die Ausgangsstoffe der Formel (VI) seien genannt:

Aceton, Methylethylketon, Methyl-isopropylketon, Methylisobutylketon, Methyl-cyclopropylketon, Benztraubensäure-methylester und -ethylester sowie Acetophenon.

Die Ausgangsstoffe der Formel (VI) sind bekannte organische Synthesechemikalien.

Das erfindungsgemäße Verfahren (h) wird unter Einsatz eines Metallierungsmittels durchgeführt. Als solche kommen vorzugsweise Alkalimetall- oder Erdalkalimetall-alkyl-Verbindungen bzw. Grignard-Verbindungen, beispielsweise Butyl-lithium oder Isopropylmagnesium-chlorid, aber auch freie Alkalimetalle oder Erdalkalimetalle, wie Lithium oder Magnesium, in Betracht.

Das erfindungsgemäße Verfahren (h) wird vorzugsweise unter Verwendung von Verdünnungsmitteln durchgeführt. Als solche kommen vorzugsweise gegen Metallierungsmittel relativ inerte organische Lösungsmittel in Betracht. Hierzu gehören insbesondere Kohlenwasserstoffe, wie Pentan, Hexan, Heptan, Octan, Cyclohexan, Petrolether, Benzin und Ligroin, sowie Ether, wie Diethylether, Dipropylether, Diisopropylether, Dibutylether, Diisobutylether, Glycoldimethylether, Diglycoldimethylether, Tetrahydrofuran und Dioxan.

Die Reaktionstemperaturen können bei dem erfindungsgemäßen Verfahren (h) in einem größeren Bereich variiert werden. Im allgemeinen arbeitet man bei Temperaturen zwischen -70 °C und +100 °C, vorzugsweise bei Temperaturen zwischen 0 °C und 80 °C.

Zur Durchführung des erfindungsgemäßen Verfahrens (h) setzt man auf 1 Mol Ausgangsverbindung der Formel (Ia) im allgemeinen zwischen 1 und 3 Mol, vorzugsweise zwischen 1 und 2 Mol, Metallierungsmittel und zwischen 0,8 und 1,5 Mol, vorzugsweise zwischen 0,9 und 1,2 Mol, Carbonylverbindung der Formel (VI) ein.

In einer bevorzugten Ausführungsform des erfindunggemäßen Verfahrens (h) wird das Metallierungsmittel in einem Verdünnungsmittel vorgelegt, das halogensubstituierte Phenoxybenzylhalogenid der Formel (Ia) eindosiert und nach abgeschlossener Umsetzung dieser Komponenten die Carbonylverbindung der Formel (VI) dazu gegeben. Nach Ende der Reaktion kann nach üblichen Methoden aufgearbeitet werden.

Die beim erfindungsgemäßen Verfahren (i) zur Herstellung von Verbindungen der Formel (I) als Ausgangsstoffe zu verwendenden Phenoxybenzylverbindungen sind durch die Formel (VII) allgemein definiert.

In Formel (VII) haben $R^1$, $R^2$, $R^3$, $R^4$, $R^5$ und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$, $R^5$ und Y angegeben wurden.

Beispiele für die Ausgangsstoffe der Formel (VII) sind in der nachstehenden Tabelle 3 aufgeführt.

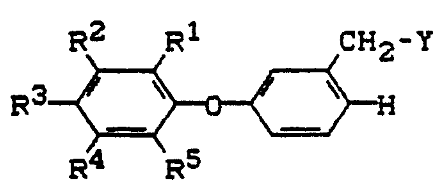

(VII)

Tabelle 3: Beispiele für die Ausgangsstoffe der Formel (VII)

| $R^1$ | $R^2$ | $R^3$ | $R^4$ | $R^5$ | Y |
|---|---|---|---|---|---|
| Cl | H | $CF_3$ | H | H | Cl |
| Cl | H | $CF_3$ | H | H | Br |
| Cl | H | $CF_3$ | H | Cl | Cl |
| Cl | H | $CF_3$ | H | Cl | Br |
| Cl | H | $CF_3$ | H | F | Cl |
| Cl | H | $CF_3$ | H | F | Br |
| Cl | H | $CF_3$ | Cl | Cl | Cl |

Tabelle 3 - Fortsetzung

| $R^1$ | $R^2$ | $R^3$ | $R^4$ | $R^5$ | Y |
|---|---|---|---|---|---|
| Cl | H | $CF_3$ | F | Cl | Cl |
| Cl | H | $CF_3$ | H | H | CN |
| Cl | H | $CF_3$ | H | Cl | CN |
| Cl | H | $CF_3$ | H | F | CN |
| Cl | H | $CF_3$ | Cl | Cl | CN |
| Cl | H | $CF_3$ | F | Cl | CN |
| Cl | H | $CF_3$ | H | H | $CH(COOH)_2$ |
| Cl | H | $CF_3$ | H | Cl | $CH(COOH)_2$ |
| Cl | H | $CF_3$ | H | F | $CH(COOH)_2$ |
| Cl | H | $CF_3$ | Cl | Cl | $CH(COOH)_2$ |
| Cl | H | $CF_3$ | F | Cl | $CH(COOH)_2$ |
| Cl | H | $CF_3$ | H | H | $CH(COOH)_2$ |
| Cl | H | $CF_3$ | H | Cl | $CH(COOCH_3)_2$ |
| Cl | H | $CF_3$ | H | F | $CH(COOCH_3)_2$ |
| Cl | H | $CF_3$ | Cl | Cl | $CH(COOCH_3)_2$ |
| Cl | H | $CF_3$ | F | Cl | $CH(COOCH_3)_2$ |
| Cl | H | $CF_3$ | H | H | $CH(COOC_2H_5)_2$ |
| Cl | H | $CF_3$ | H | Cl | $CH(COOC_2H_5)_2$ |
| Cl | H | $CF_3$ | Cl | Cl | $CH(COOC_2H_5)_2$ |
| Cl | H | $CF_3$ | H | F | $CH(COOC_2H_5)_2$ |
| Cl | H | $CF_3$ | F | Cl | $CH(COOC_2H_5)_2$ |

Tabelle 3 - Fortsetzung

| $R^1$ | $R^2$ | $R^3$ | $R^4$ | $R^5$ | Y |
|---|---|---|---|---|---|
| Cl | H | $CF_3$ | H | H | $-\overset{O}{\overset{\|}{P}}(OCH_3)_2$ |
| Cl | H | $CF_3$ | H | Cl | $-\overset{O}{\overset{\|}{P}}(OCH_3)_2$ |
| Cl | H | $CF_3$ | H | F | $-\overset{O}{\overset{\|}{P}}(OCH_3)_2$ |
| Cl | H | $CF_3$ | Cl | Cl | $-\overset{O}{\overset{\|}{P}}(OCH_3)_2$ |
| Cl | H | $CF_3$ | F | Cl | $-\overset{O}{\overset{\|}{P}}(OCH_3)_2$ |
| Cl | H | $CF_3$ | H | H | $-\overset{O}{\overset{\|}{P}}(OC_2H_5)_2$ |
| Cl | H | $CF_3$ | H | Cl | $-\overset{O}{\overset{\|}{P}}(OC_2H_5)_2$ |
| Cl | H | $CF_3$ | H | F | $-\overset{O}{\overset{\|}{P}}(OC_2H_5)_2$ |
| Cl | H | $CF_3$ | Cl | Cl | $-\overset{O}{\overset{\|}{P}}(OC_2H_5)_2$ |
| Cl | H | $CF_3$ | F | Cl | $-\overset{O}{\overset{\|}{P}}(OC_2H_5)_2$ |
| Cl | H | $CF_3$ | H | H | -COOH |

Tabelle 3 - Fortsetzung

| $R^1$ | $R^2$ | $R^3$ | $R^4$ | $R^5$ | Y |
|---|---|---|---|---|---|
| Cl | H | $CF_3$ | H | Cl | -COOH |
| Cl | H | $CF_3$ | H | F | -COOH |
| Cl | H | $CF_3$ | Cl | Cl | -COOH |
| Cl | H | $CF_3$ | F | Cl | -COOH |
| Cl | H | $CF_3$ | H | H | $-COOCH_3$ |
| Cl | H | $CF_3$ | H | Cl | $-COOCH_3$ |
| Cl | H | $CF_3$ | H | F | $-COOCH_3$ |
| Cl | H | $CF_3$ | Cl | Cl | $-COOCH_3$ |
| Cl | H | $CF_3$ | F | Cl | $-COOCH_3$ |
| Cl | H | $CF_3$ | H | H | $-COOC_2H_5$ |
| Cl | H | $CF_3$ | H | Cl | $-COOC_2H_5$ |
| Cl | H | $CF_3$ | H | F | $-COOC_2H_5$ |
| Cl | H | $CF_3$ | Cl | Cl | $-COOC_2H_5$ |
| Cl | H | $CF_3$ | F | Cl | $-COOC_2H_5$ |

Die Ausgangsstoffe der Formel (VII) sind bekannt und/oder können analog zu den erfindungsgemäßen Verfahren (a) bis (h) hergestellt werden.

Das erfindungsgemäße Verfahren (i) wird unter Einsatz von Halogen durchgeführt. Vorzugsweise werden Brom oder Chlor eingesetzt.

Verfahren (i) wird gegebenenfalls unter Verwendung von Katalysatoren durchgeührt. Als solche kommen vorzugsweise saure bzw. elektrophile Halogenverbindungen, wie z. B. Hydrogenchlorid, Hydrogenbromid, Aluminiumchlorid, Aluminiumbromid, Eisen(III)-chlorid oder Eisen(III)-bromid in Betracht.

Das erfindungsgemäße Verfahren (i) wird vorzugsweise unter Verwendung von Verdünnungsmitteln durchgeführt. Es kommen vor allem diejenigen organischen Solventien in Betracht, die bereits oben für Verfahren (b) angegeben wurden, daneben jedoch auch Essigsäure und/oder Wasser.

Die Reaktionstemperaturen können bei dem erfindungsgemäßen Verfahren (i) in einem größeren Bereich variiert werden. Im allgemeinen arbeitet man bei Temperaturen zwischen 0 °C und 150 °C, vorzugsweise bei Temperaturen zwischen 20 °C und 120 °C.

Verfahren (i) wird im allgemeinen bei Normaldruck durchgeführt.

Zur Durchführung des erfindungsgemäßen Verfahrens (i) setzt man je Mol Ausgangsverbindung der Formel (VII) im allgemeinen zwischen 1 und 5 Mol, vorzugsweise zwischen 1 und 3 Mol, Halogen ein.

Vorzugsweise wird die Ausgangsverbindung der Formel (VII) in einem Verdünnungsmittel vorgelegt und das Halogen wird langsam - gegebenenfalls bei erhöhter Temperatur - eindosiert. Das Reaktionsgemisch wird bis zum Ende der Umsetzung gerührt und anschließend eingeengt. Der Rückstand wird in einem mit Wasser praktisch nicht mischbaren organischen Lösungsmittel, wie z. B. Methylenchlorid, gelöst; diese Lösung wird mit Wasser gewaschen, mit einem üblichen Trockenmittel, wie z. B. Natriumsulfat, getrocknet und filtriert. Das Filtrat wird eingeengt, der Rückstand durch Verreiben mit einem geeigneten Lösungsmittel, wie z. B. Petrolether, zur Kristallisation gebracht und das kristalline Produkt wird durch Absaugen isoliert.

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z. B. Wasser, Methanol, Ethanol oder Aceton, und Zugabe einer geeigneten Säure bzw. Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u>

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u>

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u>

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u>

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich insbesondere zur selektiven Bekämpfung von dikotylen Unkräutern in monokotylen Kulturen, vor allem im Nachauflauf-Verfahren.

Einige der erfindungsgemäßen Verbindungen der Formel (I) zeigen auch fungizide Wirkung, beispielsweise gegen Pyricularia oryzae an Reis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol

oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. Mischungen mit 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-[4-(2,4-Dichlorphenoxy)-phenoxy]-propionsäure, deren Methyl-oder deren Ethylester (DICLOFOP); 4-Amino-6-t-butyl-3-ethylthio-1,2,4-triazin-5-(4H)-on (ETHIOZIN); 2-{4-[(6-Chlor-2-benzoxazolyl)-oxy]-phenoxy}-propansäure, deren Methyl-oder deren Ethylester (FENOXAPROP); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON) und 3,5,6-Trichlor-2-pyridyloxyessigsäure (TRICLOPYR) sind ebenfalls möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

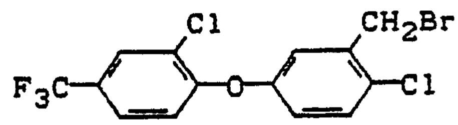

(Verfahren (a))

38,5 g (0,12 Mol) 2-Chlor-5-(2-chlor-4-trifluormethylphenoxy)-toluol werden in 300 ml Tetrachlorkohlenstoff gelöst; dazu werden 23,1 g (0,16 Mol) N-Brom-succinimid und 1 g Azo-bis-isobutyronitril gegeben. Die Reaktionsmischung wird 24 Stunden unter Rückfluß zum Sieden erhitzt, filtriert und das Filtrat eingeengt. Der Rückstand wird mit n-Hexan als Laufmittel über eine Kieselgelsäule chromatografiert.

Man erhält 19,3g (40% der Theorie) 2-Chlor-5-(2-Chlor-4-trifluormethyl-phenoxy)-benzylbromid als gelbliches Öl.

m/e = 398 / 400 / 402

$^1$H-NMR (300 MHz, $CDCl_3$/TMS) $-CH_2-Br$, $\delta$ = 4,55 ppm (s)

### Beispiel 2

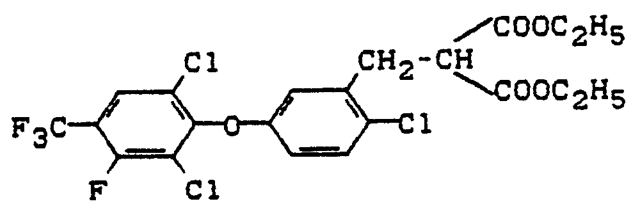

(Verfahren (b))

2,4 g (0,015 Mol) Malonsäurediethylester werden in 20 ml Ethanol gelöst und 0,81 g (0,015 Mol) Natriummethanolat dazu gegeben. Das Gemisch wird 30 Minuten gerührt und dann zur Trockene eingedampft. Das so hergestellte Natriumsalz des Malonsäurediethylesters gibt man zu einer Lösung von 5,4 g (0,012 Mol) 2-Chlor-5-(2,6-dichlor-3-fluor-4-trifluormethyl-phenoxy)-benzylbromid in 100 ml Acetonitril und erhitzt das Reaktionsgemisch 4,5 Stunden unter Rückfluß. Danach wird der Ansatz eingeengt, der Rückstand in Methylenchlorid aufgenommen und mit Wasser gewaschen. Die Methylenchloridphase wird über Natriumsulfat getrocknet, filtriert und das Filtrat unter vermindertem Druck eingeengt. Das obige Produkt läßt sich durch Zugabe von n-Hexan bei -20 °C auskristallisieren und absaugen.

Man erhält 2,8 g (44 % der Theorie) 2-Chlor-5-(2,6-dichlor-3-fluor-4-trifluormethyl-phenoxy)-benzylmalonsäurediethylester vom Schmelzpunkt 85 °C - 87 °C.

### Beispiel 3

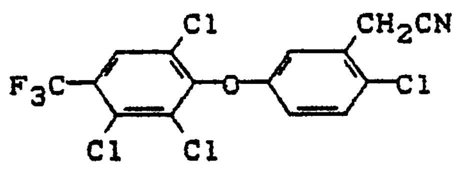

(Verfahren (b))

9,4 g (0,02 Mol) 2-Chlor-5-(2,3,6-trichlor-4-trifluormethyl-phenoxy)-benzylbromid werden in 80 ml Ethanol und 20 ml Wasser mit 1,5 g (0,03 Mol) Natriumcyanid 12 Stunden unter Rückfluß zum Sieden erhitzt. Das Reaktionsgemisch wird danach eingeengt und der Rückstand in Methylenchlorid aufgenommen. Die Methylenchloridlösung wird mit Wasser gewaschen, über Natriumsulfat getrocknet, filtriert und eingedampft. Der ölige Rückstand läßt sich mit n-Hexan auskristallisieren.

Man erhält 4,3 g (52 % der Theorie) 2-Chlor-5-(2,3,6-trichlor-4-trifluormethyl-phenoxy)-benzyl-cyanid in Form farbloser Kristalle vom Schmelzpunkt 106 °C - 110 °C.

Beispiel 4

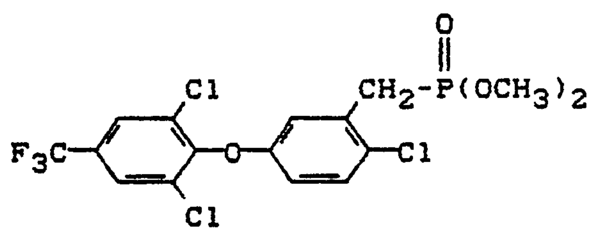

(Verfahren (c))

Eine Mischung aus 4,34 g (0,01 Mol) 2-Chlor-5-(2,6-dichlor-4-trifluormethyl-phenoxy)-benzylbromid und 30 ml (0,25 Mol)Trimethylphosphit wird 48 Stunden auf 110 °C erhitzt. Anschließend wird überschüssiges Trimethylphosphit unter vermindertem Druck abdestilliert und der Rückstand mit Methylenchlorid/Wasser geschüttelt. Von der organischen Phase wird das Lösungsmittel unter vermindertem Druck sorgfältig abdestilliert.

Man erhält 3,5 g (76 % der Theorie) 2-Chlor-5-(2,6-dichlor-4-trifluormethyl-phenoxy)-benzylphosphonsäure-dimethylester vom Schmelzpunkt 65 °C.

Beispiel 5

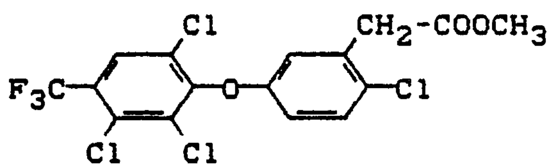

(Verfahren (d))

4,15 g (0,01 Mol) 2-Chlor-5-(2,3,6-trichlor-4-trifluormethyl-phenoxy)-benzylcyanid werden in einem Gemisch von 40 ml Diethylether und 40 ml Methanol gelöst, die Lösung auf 0 °C bis 5 °C abgekühlt und mit

Hydrogenchlorid gesättigt. Man rührt noch 12 Stunden bei Raumtemperatur, engt das Reaktionsgemisch ein und nimmt den Rückstand in Methylenchlorid auf. Die Methylenchloridlösung wird mit Wasser gewaschen, über Natriumsulfat getrocknet, filtriert und eingedampdft. Der ölige Rückstand wird mit Petrolether auskristallisiert, die Kristalle abgesaugt und an der Luft getrocknet.

Man erhält 2,8 g (62 % der Theorie) 2-Chlor-5-(2,3,6-trichlor-4-trifluormethyl-phenoxy)-phenylessigsäure-methylester in Form farbloser Kristalle vom Schmelzpunkt 112 °C - 115 °C.

Beispiel 6

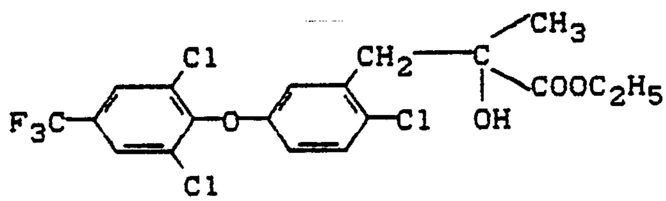

(Verfahren (h))

Eine Lösung von 8,7 g (0,02 Mol) 2-Chlor-5-(2,6-dichlor-4-trifluormethyl-phenoxy)-benzylbromid in 40 ml Diethylether wird tropfenweise zu 0,55 g (0,023 Mol) Magnesium in 30 ml Diethylether gegeben und das Gemisch wird 2 Stunden unter Rückfluß zum Sieden erhitzt. Dann werden bei 20 °C 2,3 g (0,02 Mol) Brenztraubensäureethylester dazu gegeben. Man läßt die Mischung 8 Stunden stehen und gibt dann 50 ml einer 10 %igen Ammoniumchlorid-Lösung dazu. Die organische Phase wird abgetrennt, mit Magnesiumsulfat getrocknet und filtriert. Vom Filtrat wird das Lösungsmittel im Wasserstrahlvakuum sorgfältig abdestilliert.

Man erhält 8,5 g (90 % der Theorie) 1-Hydroxy-1-methyl-2-(2-chlor-5-(2,6-dichlor-4-trifluormethyl-phenoxy)-phenyl)-propionsäure-ethylester als öligen Rückstand.

$^1$H-NMR ($CDCl_3$, δ): 1,20 (t, $OCH_2C\underline{H}_3$); 3,12 (q, 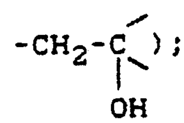 );

7,63 (s, 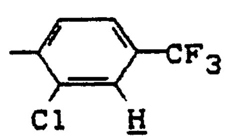 ).

Analog zu den Beispielen 1 bis 6 sowie nach der allgemeinen Beschreibung der erfindungsgemäßen Herstellungsverfahren können die in der nachstehenden Tabelle 4 aufgeführten Verbindungen der Formel (I) erhalten werden.

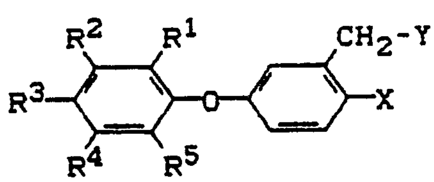 (I)

**Tabelle 4**: Herstellungsbeispiele für die Verbindungen der Formel (I)

| Beisp.-Nr. | $R^1$ | $R^2$ | $R^3$ | $R^4$ | $R^5$ | X | Y | Schmelzpunkt (°C) |
|---|---|---|---|---|---|---|---|---|
| 7 | Cl | H | $CF_3$ | H | Cl | Cl | -COOH | 153 |
| 8 | Cl | H | $CF_3$ | H | Cl | Cl | 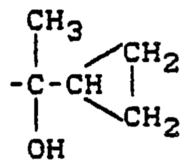 | |
| 9 | Cl | H | $CF_3$ | H | Cl | Cl | $-COOC_2H_5$ | |
| 10 | Cl | H | $CF_3$ | H | Cl | Cl | 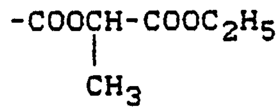 | |
| 11 | Cl | H | $CF_3$ | H | Cl | Cl | 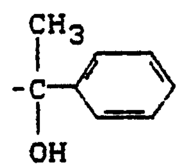 | |
| 12 | Cl | H | $CF_3$ | H | Cl | Cl | -CN | |
| 13 | Cl | H | $CF_3$ | H | Cl | Cl | $-N_3$ | |

Tabelle 4 - Fortsetzung

| Beisp.-Nr. | $R^1$ | $R^2$ | $R^3$ | $R^4$ | $R^5$ | X | Y | Schmelzpunkt (°C) |
|---|---|---|---|---|---|---|---|---|
| 14 | Cl | H | $CF_3$ | H | Cl | Cl | $-NH_2$ | 250 |
| 15 | Cl | H | $CF_3$ | H | Cl | Cl | $-NHCOOCH_3$ | 122 |
| 16 | Cl | H | $CF_3$ | H | Cl | Cl | 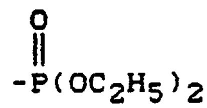 | |
| 17 | Cl | H | $CF_3$ | H | Cl | Cl | $-COOCH_2COOC_2H_5$ | |
| 18 | Cl | H | $CF_3$ | H | Cl | Cl | $-CONH_2$ | 180 |
| 19 | Cl | H | $CF_3$ | H | Cl | Cl | -SCN | 98 |
| 20 | Cl | H | $CF_3$ | H | Cl | Cl | $-N(COCH_3)_2$ | 120 |
| 21 | Cl | H | $CF_3$ | H | Cl | Cl | $-NHCOCH_3$ | 140 |
| 22 | Cl | H | $CF_3$ | H | Cl | Cl | $-N(CH_3)_2$ | 190 |
| 23 | Cl | H | $CF_3$ | H | Cl | Cl | $-NHCH_3$ | |
| 24 | Cl | H | $CF_3$ | H | Cl | Cl | $-NH-COOC_2H_5$ | 152 |
| 25 | Cl | H | $CF_3$ | H | Cl | Cl | 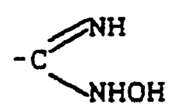 | 162 |
| 26 | Cl | H | $CF_3$ | H | Cl | Cl | $-NHSO_2CH_3$ | 126 |
| 27 | Cl | H | $CF_3$ | H | Cl | Cl | $-COOCH_3$ | 95 |
| 28 | Cl | H | $CF_3$ | H | Cl | Cl | 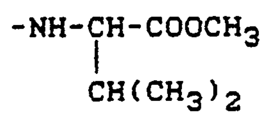 | |
| 29 | Cl | H | $CF_3$ | H | Cl | Cl | $-NH=CH-N(CH_3)_2$ | |
| 30 | Cl | H | $CF_3$ | H | Cl | Cl | $-NHCOCF_3$ | 122 |

Tabelle 4 - Fortsetzung

| Beisp.-Nr. | $R^1$ | $R^2$ | $R^3$ | $R^4$ | $R^5$ | X | Y | Schmelzpunkt (°C) |
|---|---|---|---|---|---|---|---|---|
| 31 | Cl | H | $CF_3$ | H | Cl | Cl | Cl | 42 |
| 32 | Cl | H | $CF_3$ | H | Cl | Cl | 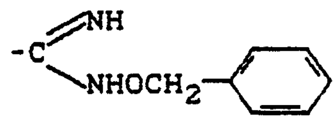 | |
| 33 | Cl | H | $CF_3$ | H | Cl | Cl | $-CONHSO_2CH_3$ | 223 |
| 34 | Cl | H | $CF_3$ | H | H | Cl | -NHCN | |
| 35 | Cl | H | $CF_3$ | Cl | Cl | Cl | Br | |
| 36 | Cl | H | $CF_3$ | Cl | Cl | Cl | -CN | 93 |
| 37 | Cl | H | $CF_3$ | H | Cl | Cl | $-CH(COOC_2H_5)_2$ | 91 |
| 38 | Cl | H | $CF_3$ | H | H | Cl | $-CH(COOC_2H_5)_2$ | |
| 39 | Cl | H | $CF_3$ | H | H | Cl | $-COOCH_3$ | 74 |
| 40 | Cl | H | $CF_3$ | F | Cl | Cl | Br | 80 |
| 41 | Cl | H | $CF_3$ | H | Cl | Cl | $-CH(COQH)_2$ | 153 |
| 42 | Cl | H | $CF_3$ | Cl | Cl | Cl | $-CH(COOC_2H_5)_2$ | 85 |
| 43 | $CF_3$ | H | $CF_3$ | H | H | Cl | $-CH(COOC_2H_5)_2$ | 40 |
| 44 | Cl | H | $CF_3$ | Cl | Cl | Br | Br | 83 |
| 45 | Cl | H | $CF_3$ | Cl | Cl | Br | $-CH(COOC_2H_5)_2$ | 89 |
| 46 | Cl | H | $CF_3$ | Cl | Cl | Br | 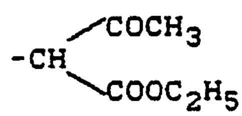 | |
| 47 | Cl | H | $CF_3$ | Cl | Cl | Br | $-COOCH_3$ | 162 |
| 48 | Cl | G | $CF_3$ | H | H | Br | $-COOC_2H_5$ | |

Tabelle 4 - Fortsetzung

| Beisp.-Nr. | $R^1$ | $R^2$ | $R^3$ | $R^4$ | $R^5$ | X | Y | Schmelzpunkt (°C) |
|---|---|---|---|---|---|---|---|---|
| 49 | Cl | H | $CF_3$ | H | H | Br | $-CH(COOC_2H_5)_2$ | |
| 50 | Cl | H | $CF_3$ | H | H | Cl | $-CH(COOH)_2$ | 127 |
| 51 | Cl | H | $CF_3$ | Cl | Cl | Cl | -SCN | 84 |
| 52 | Cl | H | $CF_3$ | H | Cl | Cl | 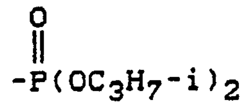 | |
| 53 | Cl | H | $CF_3$ | H | H | Cl | -SCN | |
| 54 | Cl | H | $CF_3$ | H | H | Cl | -COOH | 70 |
| 55 | Cl | H | $CF_3$ | H | H | Cl | 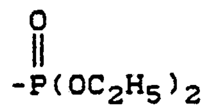 | |
| 56 | Cl | H | $CF_3$ | H | Cl | Cl | 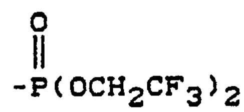 | 98 |
| 57 | Cl | H | $CF_3$ | H | H | Cl | $-NH_2$ | 212 |
| 58 | Cl | H | $CF_3$ | H | H | Cl | $-NH-OCH_3$ | |
| 59 | Cl | H | $CF_3$ | H | H | Cl | 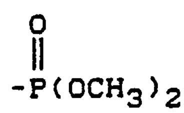 | |
| 60 | Cl | H | $CF_3$ | Cl | Cl | Cl | 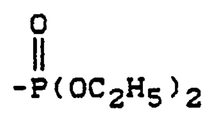 | |
| 61 | Cl | H | $CF_3$ | H | Cl | Cl | 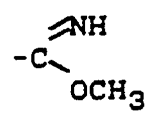 x HCl | 155 |
| 62 | Cl | H | $CF_3$ | H | F | Cl | Br | |

<u>Tabelle 4</u> - Fortsetzung

| Beisp.-Nr. | $R^1$ | $R^2$ | $R^3$ | $R^4$ | $R^5$ | X | Y | Schmelzpunkt (°C) |
|---|---|---|---|---|---|---|---|---|
| 63 | Cl | H | $CF_3$ | Cl | Cl | Cl | $-\overset{O}{\overset{\|}{P}}(OCH_3)_2$ | 72 |
| 64 | Cl | H | $CF_3$ | H | Cl | Cl | $-\overset{O}{\overset{\|}{P}}(OC_4H_9-i)$ | |
| 65 | Cl | H | $CF_3$ | H | Cl | Cl | $-NH-OCH_3$ | 71 |
| 66 | Cl | H | $CF_3$ | H | F | Cl | $-\overset{O}{\overset{\|}{P}}(OCH_3)_2$ | |
| 67 | Cl | H | $CF_3$ | H | F | Cl | $-CN$ | |
| 68 | Cl | H | $CF_3$ | H | Cl | Cl | $-NH-O-CH_2COOC_2H_5$ | 33 |
| 69 | Cl | H | $CF_3$ | H | F | Cl | $-NH-O-CH_2COOC_2H_5$ | |
| 70 | Cl | H | $CF_3$ | H | Cl | Cl | $-NH-O-\underset{CH_3}{\underset{\|}{C}}HCOOC_2H_5$ | |
| 71 | Cl | H | $CF_3$ | H | F | Cl | $-NH-O-\underset{CH_3}{\underset{\|}{C}}HCOOCH_3$ | |
| 72 | Cl | H | $CF_3$ | H | F | Cl | $-NH-O-\underset{CH_3}{\underset{\|}{C}}HCOOC_2H_5$ | |
| 73 | Cl | H | $CF_3$ | H | Cl | Cl | $-NH-O-CH_2COOCH_3$ | |
| 74 | Cl | H | $CF_3$ | H | F | Cl | $-NH-O-CH_2COOCH_3$ | |
| 75 | Cl | H | $CF_3$ | H | H | Cl | $-NH-O-CH_2COOC_2H_5$ | |
| 76 | Cl | H | $CF_3$ | H | H | Cl | $-N\begin{matrix}OCH_3\\CH_2COOC_2H_5\end{matrix}$ | |

Tabelle 4 - Fortsetzung

| Beisp.-Nr. | $R^1$ | $R^2$ | $R^3$ | $R^4$ | $R^5$ | X | Y | Schmelzpunkt (°C) |
|---|---|---|---|---|---|---|---|---|
| 77 | Cl | H | $CF_3$ | H | Cl | Cl | $-NH-O-\underset{CH_3}{\underset{\vert}{C}}HCOOCH_3$ | |
| 78 | Cl | H | $CF_3$ | H | Cl | Cl | $-NHCH_2CH_2COOCH_3$ | |
| 79 | Cl | H | $CF_3$ | H | F | Cl | $-NH-O-CH_2COOC_4H_9$ | |
| 80 | Cl | H | $CF_3$ | H | Cl | Cl | $-NH-O-CH_2-$ 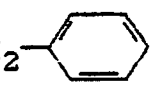 | 78 |
| 81 | Cl | H | $CF_3$ | H | F | Cl | $-\overset{O}{\overset{\Vert}{P}}(OC_2H_5)$ | |
| 82 | Cl | H | $CF_3$ | H | F | Cl | $-NH_2$ | 220 |
| 83 | Cl | H | $CF_3$ | H | H | Cl | $-NH-CO-CH_3$ | 99 |
| 84 | Cl | H | $CF_3$ | Cl | Cl | Br | -CN | 106 |

Anwendungsbeispiele

In den folgenden Anwendungsbeispielen wird die nachstehend aufgeführte Verbindung als Vergleichssubstanz herangezogen:

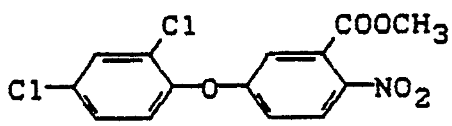

(A)

3-(2,4-Dichlor-phenoxy)-6-nitrobenzoesäure-methylester

(bekannt aus US-PS 36 52 645 und US-PS 3 776 715).

Beispiel A

Post-emergence-Test

Lösungsmittel: 5 Gewichtsteile Aceton Emulgator: 1 Gewichtsteil Alkylarylpolyglykolether

Zur Herstellung einer zweckmäßigen Wirkstoffzubereitung vermischt man 1 Gewichtsteil Wirkstoff mit

der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

| | | |
|---|---|---|
| 0 % | = | keine Wirkung (wie unbehandelte Kontrolle) |
| 100 % | = | totale Vernichtung |

In diesem Test zeigen beispielsweise die Verbindungen gemäß den Herstellungsbeispielen (1), (6), (7), (8), (9), (10), (12), (14), (15), (16), (17), (19), (35) und (37) bei guter Selektivität in Weizen erheblich stärkere Wirkung gegen Unkräuter, wie z. B. Datura, Galium, Polygonum, Portulak, Sinapis und Solanum, als die Vergleichssubstanz (A).

## Ansprüche

1. Halogensubstituierte Phenoxybenzylverbindungen der allgemeinen Formel (I)

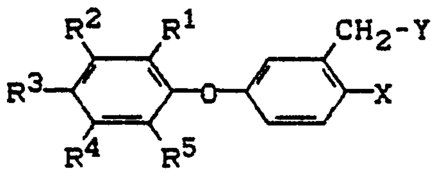

(I)

in welcher

$R^1$ für Wasserstoff, Halogen, Cyano oder Trifluormethyl steht,

$R^2$ für Wasserstoff oder Halogen steht,

$R^3$ für Halogen, Cyano, Trifluormethyl, Trifluormethoxy oder Trifluormethylsulfonyl steht,

$R^4$ für Wasserstoff oder Halogen steht,

$R^5$ für Wasserstoff oder Halogen steht,

X für Halogen steht und

Y für Halogen, Cyano, Azido, Thiocyanato, Cyanamino, oder für eine der nachstehenden Gruppierungen

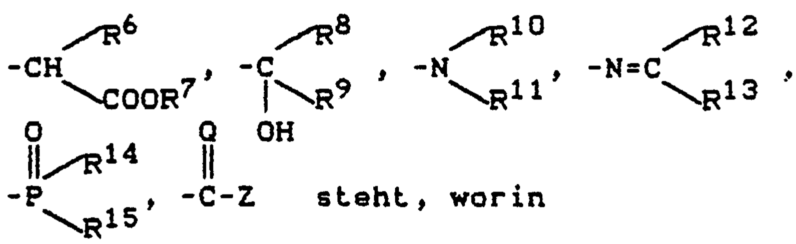

steht, worin

$R^6$ für Cyano, Carboxy, für jeweils gegebenenfalls durch Halogen substituiertes Alkylcarbonyl oder Alkoxycarbonyl steht,

$R^7$ für Wasserstoff oder Alkyl steht,

$R^8$ für Wasserstoff oder Alkyl steht,

$R^9$ für Alkyl, Cycloalkyl, Alkoxycarbonyl oder gegebenenfalls durch Halogen, Alkyl und/oder Alkoxy substituiertes Phenyl steht,

$R^{10}$ für Wasserstoff, für gegebenenfalls durch Halogen, Hydroxy, Alkoxy oder Alkoxycarbonyl substituiertes Alkyl, für Alkenyl, Alkinyl oder Benzyl, für gegebenenfalls durch Halogen oder Alkoxy substituiertes Alkylcarbonyl, für Alkoxycarbonyl, Benzyloxycarbonyl oder Phenoxycarbonyl, für gegebenenfalls durch Halogen substituiertes Alkylsulfonyl, für jeweils gegebenenfalls durch Halogen, Nitro, Alkyl, Halogenalkyl,

Alkoxy, Halogenalkoxy, Alkylthio, Alkylsulfinyl, Alkylsulfonyl, Dialkylaminosulfonyl und/oder Alkoxycarbonyl substituiertes Phenylsulfonyl oder Benzylsulfonyl steht,

$R^{11}$ für Hydroxy, für gegebenenfalls durch Alkoxycarbonyl substituiertes Alkoxy, für Alkenyloxy, Benzyloxy, für jeweils gegebenenfalls durch Halogen, Cyano, Nitro, Alkyl, Halogenalkyl, Alkoxy, Halogenalkoxy, Alkylthio, Halogenalkylthio, Alkylsulfinyl, Alkylsulfonyl, Dialkylaminosulfonyl, Alkoxycarbonyl und/oder Alkylendioxy substituiertes Phenyl oder Naphthyl steht, oder $R^{11}$ weiterhin eine der oben für $R^{10}$ angegebenen Bedeutungen hat oder

$R^{11}$ zusammen mit $R^{10}$ für gegebenenfalls verzweigtes und gegebenenfalls durch Sauerstoff unterbrochenes Alkandiyl steht,

$R^{12}$ für Wasserstoff oder Alkyl steht,

$R^{13}$ für Alkyl, Cycloalkyl, Aralkyl, Alkoxy, Dialkylamino oder für gegebenenfalls durch Halogen, Alkyl und/oder Alkoxy substituiertes Phenyl steht

$R^{14}$ für jeweils gegebenenfalls duroh Halogen substituiertes Alkyl oder Alkoxy steht,

$R^{15}$ für jeweils gegebenenfalls durch Halogen substituiertes Alkyl oder Alkoxy steht,

Q für Sauerstoff, Schwefel oder NH steht und

Z für Halogen, Cyano, Azido, Thiocyanato, Cyanamino, Hydrazino, Alkylhydrazino, Benzylhydrazino, gegebenenfalls durch Halogen, Alkyl, Halogenalkyl und/oder Alkoxy substituiertes Phenylhydrazino, für Alkylcarbonylhydrazino oder Alkoxycarbonylhydrazino, für gegebenenfalls durch Halogen, Nitro und/oder Alkyl substituiertes Benzoylhydrazino, für gegebenenfalls durch Halogen substituiertes Alkylsulfonylhydrazino, für gegebenenfalls durch Halogen, Cyano, Nitro, Alkyl, Halogenalkyl, Alkoxy, Halogenalkoxy, Alkylthio, Alkylsulfinyl, Alkylsulfonyl, Dialkylaminosulfonyl und/oder Alkoxycarbonyl substituiertes Phenylsulfonylhydrazino, für Alkylthio, Benzylthio, gegebenenfalls durch Halogen, Alkyl und/oder Alkoxy substituiertes Phenylthio, für Naphthylthio oder Alkoxycarbonylalkylthio oder für eine der nachstehenden Gruppierungen

$-O-R^{16}$, 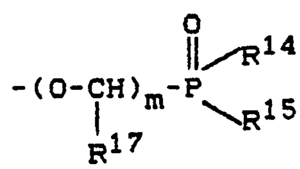, 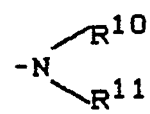,

$-O-(CH_2)_n-R^{18}$ steht, worin

m für die Zahlen 0 oder 1 steht,

n für die Zahlen 0, 1 oder 2 steht,

$R^{16}$ für Wasserstoff, für gegebenenfalls durch Halogen, Alkoxy, Alkylthio, Alkylsulfinyl, Alkylsulfonyl, Aralkoxy, Aralkylthio, Alkoxycarbonyl, Alkylaminocarbonyl, Azolyl oder Trialkylsilyl substituiertes Alkyl, für jeweils gegebenenfalls durch Halogen substituiertes Alkenyl oder Alkinyl, für jeweils gegebenenfalls durch Halogen und/oder Alkyl substituiertes Cycloalkyl oder Cycloalkylalkyl, für gegebenenfalls durch Halogen, Alkyl, Alkoxy und/oder Alkoxycarbonyl substituiertes Benzyl, für jeweils gegebenenfalls durch Halogen, Alkyl, Halogenalkyl, Alkoxy und/oder Halogenalkoxy substituiertes Phenyl oder Naphthyl, oder für ein Ammonium-, Alkylammonium-, Alkalimetall- oder Erdalkalimetall-Äquivalent steht,

$R^{17}$ für Wasserstoff, Alkyl, jeweils gegebenenfalls durch Halogen, Alkyl, Halogenalkyl und/oder Alkoxy substi tuiertes Phenyl, Furyl, Thienyl oder Pyridyl steht,

$R^{18}$ für eine gegebenenfalls durch Halogen und/oder Alkyl substituierte heterocyclische Gruppierung aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht, sowie

$R^{10}$, $R^{11}$, $R^{14}$ und $R^{15}$ die oben angegebenen Bedeutungen haben,

sowie Salze von Verbindungen der Formel (I), wobei die Verbindungen 2- Chlor-5-(2-chlor-4-trifluormethyl-phenoxy)-benzylchlorid und 2-Chlor-5-(2-chlor-4-trifluormethyl- phenoxy)-benzylcyanid und 2-Brom-5-(2-chlor-4- trifluormethyl-phenoxy)-benzylbromid ausgenommen sind.

2. Halogensubstituierte Phenoxybenzylverbindungen der Formel (I) gemäß Anspruch 1, in welcher

$R^1$ für Wasserstoff, Fluor, Chlor, oder Brom, Cyano oder Trifluormethyl steht,

$R^2$ für Wasserstoff, Fluor, Chlor oder Brom steht,

$R^3$ für Fluor, Chlor, Brom, Cyano, Trifluormethyl, Trifluormethoxy oder Trifluormethylsulfonyl steht,

$R^4$ für Wasserstoff, Fluor, Chlor oder Brom steht,

$R^5$ für Wasserstoff, Fluor, Chlor oder Brom steht,

X für Fluor, Chlor oder Brom steht und

Y für Fluor, Chlor, Brom, Iod, Cyano, Azido, Thiocyanato, Cyanamino oder für eine der nachstehenden Gruppierungen

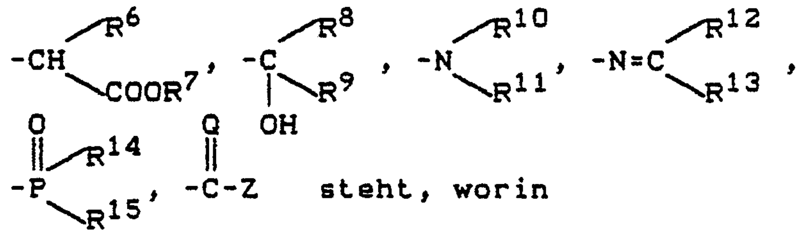

steht, worin

$R^6$ für Cyano, Carboxy, gegebenenfalls durch Fluor und/oder Chlor substituiertes $C_1$-$C_4$-Alkyl-carbonyl oder $C_1$-$C_4$-Alkoxy-carbonyl steht,

$R^7$ für Wasserstoff oder $C_1$-$C_4$-Alkyl steht,

$R^8$ für Wasserstoff oder $C_1$-$C_4$-Alkyl steht,

$R^9$ für $C_1$-$C_4$-Alkyl, $C_3$-$C_6$-Cycloalkyl, $C_1$-$C_4$-Alkoxy-carbonyl oder gegebenenfalls durch Fluor, Chlor, Brom, $C_1$-$C_4$-Alkyl und/oder $C_1$-$C_4$-Alkoxy substituiertes Phenyl steht,

$R^{10}$ für Wasserstoff, gegebenenfalls durch Fluor, Chlor, Hydroxy, $C_1$-$C_4$-Alkoxy oder $C_1$-$C_4$-Alkoxy-carbonyl substituiertes $C_1$-$C_6$-Alkyl, für $C_3$-$C_6$-Alkenyl, $C_3$-$C_6$-Alkinyl oder Benzyl, für gegebenenfalls durch Fluor, Chlor, Brom oder $C_1$-$C_4$-Alkoxy substituiertes $C_1$-$C_4$-Alkyl-carbonyl, für $C_1$-$C_4$-Alkoxy-carbonyl, Benzyloxycarbonyl oder Phenoxycarbonyl, für gegebenenfalls durch Fluor und/oder Chlor substituiertes $C_1$-$C_6$-Alkylsulfonyl, für jeweils gegebenenfalls durch Fluor, Chlor, Brom, Nitro, Methyl, Trifluormethyl, $C_1$-$C_2$-Alkoxy, durch Fluor und/oder Chlor substituiertes $C_1$-$C_2$-Alkoxy, $C_1$-$C_2$-Alkylthio, $C_1$-$C_2$-Alkylsulfinyl, $C_1$-$C_2$-Alkylsulfonyl, Di-($C_1$-$C_2$-alkyl)-aminosulfonyl und/oder $C_1$-$C_2$-Alkoxy-carbonyl substituiertes Phenylsulfonyl oder Benzylsulfonyl steht,

$R^{11}$ für Hydroxy, für gegebenenfalls durch $C_1$-$C_6$-Alkoxy-carbonyl substituiertes $C_1$-$C_6$-Alkoxy, für $C_3$-$C_6$-Alkenyloxy oder Benzyl oxy, für jeweils gegebenenfalls durch Fluor, Chlor, Brom, Cyano, Nitro, $C_1$-$C_4$-Alkyl, Trifluormethyl, $C_1$-$C_4$-Alkoxy, durch Fluor und/oder Chlor substituiertes $C_1$-$C_2$-Alkoxy, $C_1$-$C_2$-Alkylthio, durch Fluor und/oder Chlor substituiertes $C_1$-$C_4$-Alkylthio, $C_1$-$C_4$-Alkylsulfinyl, $C_1$-$C_4$-Alkylsulfonyl, Di-($C_1$-$C_2$-alkyl)-aminosulfonyl, $C_1$-$C_4$-Alkoxy-carbonyl und/oder $C_1$-$C_2$-Alkylendioxy substituiertes Phenyl oder Naphthyl steht, oder $R^{11}$ weiterhin eine der oben für $R^{10}$ vorzugsweise angegebenen Bedeutungen hat oder $R^{11}$ zusammen mit $R^{10}$ für gegebenenfalls verzweigtes und gegebenenfalls durch Sauerstoff unterbrochenes $C_2$-$C_6$-Alkandiyl steht,

$R^{12}$ für Wasserstoff oder $C_1$-$C_4$-Alkyl steht,

$R^{13}$ für $C_1$-$C_4$-Alkyl, $C_3$-$C_6$-Cycloalkyl, Benzyl, $C_1$-$C_4$-Alkoxy, Di-($C_1$-$C_2$-alkyl)-amino oder für gegebenenfalls durch Fluor, Chlor, Brom, $C_1$-$C_4$-Alkyl und/oder $C_1$-$C_4$-Alkoxy substituiertes Phenyl steht,

$R^{14}$ für jeweils gegebenenfalls durch Fluor und/ oder Chlor substituiertes $C_1$-$C_4$-Alkyl, oder $C_1$-$C_4$-Alkoxy steht,

$R^{15}$ für jeweils gegebenenfalls durch Fluor und/ oder Chlor substituiertes $C_1$-$C_4$-Alkyl oder $C_1$-$C_4$-Alkoxy steht,

Q für Sauerstoff, Schwefel oder NH steht und

Z für Chlor, Cyano, Azido, Thiocyanato, Cyanamino, Hydrazino, $C_1$-$C_4$-Alkylhydrazino, Benzylhydrazino, gegebenenfalls durch Fluor, Chlor, Brom, $C_1$-$C_4$-Alkyl, Trifluormethyl und/ oder $C_1$-$C_4$-Alkoxy substituiertes Phenylhydrazino, für $C_1$-$C_4$-Alkyl-carbonylhydrazino oder $C_1$-$C_4$-Alkoxy-carbonylhydrazino, für gegebenenfalls durch Fluor, Chlor, Brom, Nitro und/oder $C_1$-$C_4$-Alkyl substituiertes Benzoylhydrazino, für gegebenenfalls durch Fluor und/oder Chlor substituiertes $C_1$-$C_4$-Alkylsulfonylhydrazino, für gegebenenfalls durch Fluor, Chlor, Brom, Cyano, Nitro, Methyl, Trifluormethyl, $C_1$-$C_2$-Alkoxy, durch Fluor und/oder Chlor substituiertes $C_1$-$C_2$-Alkoxy, $C_1$-$C_2$-Alkylthio, $C_1$-$C_2$-Alkylsulfinyl, $C_1$-$C_2$-Alkylsulfonyl, Di-($C_1$-$C_2$-alkyl)-aminosulfonyl und/oder $C_1$-$C_2$-Alkoxy-carbonyl substituiertes Phenylsulfonylhydrazino, für $C_1$-$C_4$-Alkylthio, Benzylthio, gegebenenfalls durch Fluor, Chlor, Brom, $C_1$-$C_4$-Alkyl und/oder $C_1$-$C_4$-Alkoxy substituiertes Phenylthio, für Naphthylthio oder $C_1$-$C_4$-Alkoxy-carbonyl-$C_1$-$C_2$-alkylthio oder für eine der nachstehenden Gruppierungen

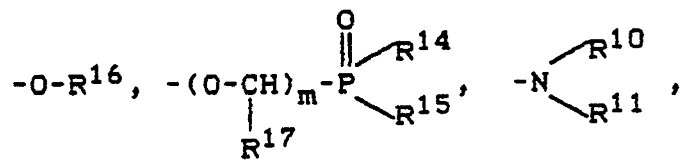

$-O-(CH_2)_n-R^{18}$ steht, worin

m für die Zahlen 0 oder 1 steht,

n für die Zahlen 0, 1 oder 2 steht,

$R^{16}$ für Wasserstoff, für gegebenenfalls durch Fluor, Chlor, $C_1$-$C_4$-Alkoxy, $C_1$-$C_4$-Alkylthio, $C_1$-$C_4$-Alkylsulfinyl, $C_1$-$C_4$-Alkylsulfonyl, Benzyloxy, Benzylthio, $C_1$-$C_4$-Alkoxy-carbonyl, $C_1$-$C_4$-Alkylamino-carbonyl, Pyrazolyl oder Trimethylsilyl substituiertes $C_1$-$C_6$-Alkyl, für jeweils gegebenenfalls durch Fluor und/oder Chlor substituiertes $C_3$-$C_4$-Alkenyl oder $C_3$-$C_4$-Alkinyl, für jeweils gegebenenfalls durch Fluor, Chlor und/oder $C_1$-$C_4$-Alkyl substituiertes $C_3$-$C_6$-Cycloalkyl oder $C_3$-$C_6$-Cycloalkyl-$C_1$-$C_3$-alkyl, für gegebenenfalls durch Fluor, Chlor, Brom, $C_1$-$C_4$-Alkyl, $C_1$-$C_4$-Alkoxy und/oder $C_1$-$C_4$-Alkoxy-carbonyl substituiertes Benzyl, für jeweils gegebenenfalls durch Fluor, Chlor, Brom, $C_1$-$C_4$-Alkyl, Trifluormethyl, $C_1$-$C_4$-Alkoxy und/oder durch Fluor und/ oder Chlor substituiertes $C_1$-$C_2$-Alkoxy substituiertes Phenyl oder Naphthyl, oder für ein Ammonium-, $C_1$-$C_4$-Alkylammonium-, Natrium-, Kalium- oder Calcium-äquivalent steht,

$R^{17}$ für Wasserstoff, $C_1$-$C_4$-Alkyl, jeweils gegebenenfalls durch Fluor, Chlor, Brom, $C_1$-$C_4$-Alkyl, Trifluormethyl, und/oder $C_1$-$C_4$-Alkoxy substituiertes Phenyl, Furyl, Thienyl oder Pyridyl steht,

$R^{18}$ für eine gegebenenfalls durch Fluor, Chlor, Brom und/oder $C_1$-$C_4$-Alkyl substituierte heterocyclische Gruppierung aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl, oder Pyrimidinyl steht, sowie

$R^{10}$, $R^{11}$, $R^{14}$, und $R^{15}$ die oben angegebenen Bedeutungen haben,

sowie deren Salze, wobei die Verbindungen 2-Chlor-5-(2-chlor-4-trifluormethylphenoxy)-benzylchlorid, 2-Chlor-5-(2-chlor-4-trifluormethylphenoxy)-benzylcyanid und 2-Brom-5-(2-chlor-4-trifluormethyl-phenoxy)-benzylbromid ausgenommen sind.

3. Halogensubstituierte Phenoxybenzylverbindungen der Formel (I) gemäß Anspruch 1, in welcher

$R^1$ für Wasserstoff, Fluor oder Chlor steht,

$R^2$ für Wasserstoff, Fluor oder Chlor steht,

$R^3$ für Trifluormethyl steht,

$R^4$ für Wasserstoff, Fluor oder Chlor steht,

$R^5$ für Wasserstoff, Fluor oder Chlor steht,

X für Chlor oder Brom steht und

Y für Chlor, Brom oder Cyano steht

mit Ausnahme der Verbindungen 2-Chlor-5-(2-chlor-4-trifluormethyl-phenoxy)-benzylchlorid, 2-Chlor-5-(2-chlor-4-trifluormethyl-phenoxy)-benzylcyanid und 2-Brom-5-(2-chlor-4-trifluormethyl-phenoxy)-benzylbromid.

4. Halogensubstituierte Phenoxybenzylverbindungen der Formel (I) gemäß Anspruch 1, in welcher

$R^1$ für Wasserstoff, Fluor oder Chlor steht,

$R^2$ für Wasserstoff, Fluor oder Chlor steht,

$R^3$ für Trifluormethyl steht,

$R^4$ für Wasserstoff, Fluor oder Chlor steht,

$R^5$ für Wasserstoff, Fluor oder Chlor steht,

X für Chlor oder Brom steht und

Y für Thiocyanato, Cyanamino oder für eine der nachstehenden Gruppierungen

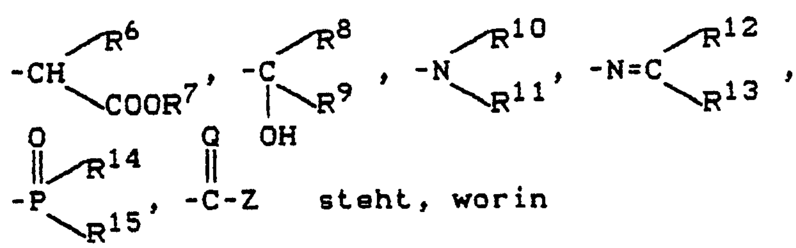

steht, worin

$R^6$ für Carboxy, Acetyl, Methoxycarbonyl oder Ethoxycarbonyl steht,

$R^7$ für Wasserstoff, Methyl oder Ethyl steht,
$R^8$ für Methyl steht,
$R^9$ für Methyl, Ethyl, Cyclopropyl, Methoxycarbonyl, Ethoxycarbonyl oder Phenyl steht,
$R^{10}$ für Wasserstoff, $C_1$-$C_4$-Alkyl, $C_1$-$C_2$-Alkoxy-carbonyl-$C_1$-$C_2$-alkyl, $C_3$-$C_4$-Alkenyl, $C_3$-$C_4$-Alkinyl oder Benzyl, für gegebenenfalls durch Fluor und/oder Chlor substituiertes $C_1$-$C_4$-Alkyl-carbonyl, für $C_1$-$C_4$-Alkoxy-carbonyl, Benzyloxycarbonyl oder Phenoxycarbonyl, für gegebenenfalls durch Fluor und/oder Chlor substituiertes $C_1$-$C_4$-Alkylsulfonyl, für jeweils gegebenenfalls durch Fluor, Chlor, Brom, Nitro, Methyl, Trifluormethyl, Methoxy, Difluormethoxy und/ oder Methoxycarbonyl substituiertes Phenylsulfonyl oder Benzylsulfonyl steht,
$R^{11}$ für Hydroxy, für gegebenenfalls durch $C_1$-$C_4$-Alkoxy-carbonyl substituiertes $C_1$-$C_4$-Alkoxy, für Allyloxy oder Benzyloxy, für jeweils gegebenenfalls durch Fluor, Chlor, Brom, Methyl, Ethyl, Trifluormethyl, Methoxy, Ethoxy, Difluormethoxy, Methylthio, Ethylthio, Trifluormethylthio, Methoxycarbonyl, oder Ethoxycarbonyl substituiertes Phenyl oder Naphthyl steht, oder $R^{11}$ weiterhin eine der oben für $R^{10}$ insbesondere angegebenen Bedeutungen hat oder
$R^{11}$ zusammen mit $R^{10}$ für gegebenenfalls verzweigtes und gegebenenfalls durch Sauerstoff unterbrochenes $C_4$-$C_5$-Alkandiyl steht,
$R^{12}$ für Wasserstoff oder Methyl steht,
$R^{13}$ für Dimethylamino steht,
$R^{14}$ für Methoxy, Ethoxy, n- oder iso-Propoxy steht,
$R^{15}$ für Methoxy, Ethoxy, n- oder iso-Propoxy steht,
Q für Sauerstoff oder NH steht und
Z für Chlor, $C_1$-$C_4$-Alkylthio, Benzylthio, $C_1$-$C_2$-Alkoxycarbonyl-$C_1$-$C_2$-alkylthio oder für eine der nachstehenden Gruppierungen

$-O-R^{16}$, 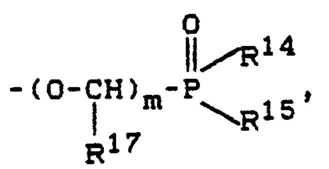, 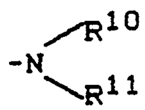,

$-O-(CH_2)_n-R^{18}$ steht, worin
m für die Zahlen 0 oder 1 steht,
n für die Zahlen, 0, 1 oder 2 steht,
$R^{16}$ für Wasserstoff, $C_1$-$C_4$-Alkyl, $C_1$-$C_2$-Alkoxy-$C_1$-$C_2$-alkyl, $C_1$-$C_2$-Alkylthio-$C_1$-$C_2$-alkyl, $C_1$-$C_2$-Alkylsulfinyl-$C_1$-$C_2$-alkyl, $C_1$-$C_2$-Alkylsulfonyl-$C_1$-$C_2$-alkyl, Benzyloxy-$C_1$-$C_3$-Alkyl, Benzylthio-$C_1$-$C_3$-alkyl, $C_1$-$C_2$-Alkoxycarbonyl-$C_1$-$C_2$-alkyl oder $C_1$-$C_2$-Alkylamino-carbonyl-$C_1$-$C_2$-alkyl steht,
$R^{17}$ für Wasserstoff, Methyl, Phenyl, Furyl, Thienyl oder Pyridyl steht,
$R^{18}$ für eine gegebenenfalls durch Chlor und/oder Methyl substituierte heterocyclische Gruppierung aus der Reihe Furyl, Tetrahydrofuryl, Thienyl, Perhydropyranyl, Oxazolyl, Thiazolyl oder Dioxolanyl steht, sowie
$R^{10}$, $R^{11}$, $R^{14}$ und $R^{15}$ die oben angegebenen Bedeutungen haben.

5. Verfahren zur Herstellung von halogensubstituierten Phenoxybenzylverbindungen der allgemeinen Formel (I)

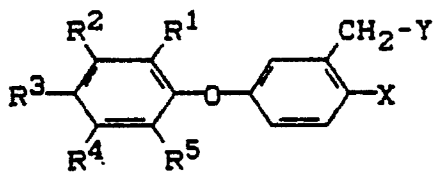 (I)

in welcher
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht,
$R^5$ für Wasserstoff oder Halogen steht,
X für Halogen steht und

Y für Halogen, Cyano, Azido, Thiocyanato, Cyanamino, oder für eine der nachstehenden Gruppierungen

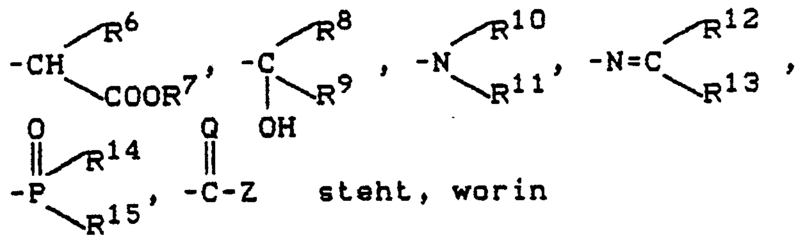

, , , , , steht, worin

$R^6$ für Cyano, Carboxy, gegebenenfalls durch Halogen substituiertes Alkylcarbonyl oder Alkoxycarbonyl steht,
$R^7$ für Wasserstoff oder Alkyl steht,
$R^8$ für Wasserstoff oder Alkyl steht,
$R^9$ für Alkyl, Cycloalkyl, Alkoxycarbonyl oder gegebenenfalls durch Halogen, Alkyl und/oder Alkoxy substituiertes Phenyl steht,
$R^{10}$ für Wasserstoff, für gegebenenfalls durch Halogen, Hydroxy, Alkoxy oder Alkoxycarbonyl substituiertes Alkyl, für Alkenyl, Alkinyl oder Benzyl, für gegebenenfalls durch Halogen oder Alkoxy substituiertes Alkylcarbonyl, für Alkoxycarbonyl, Benzyloxycarbonyl oder Phenoxycarbonyl, für gegebenenfalls durch Halogen substituiertes Alkylsulfonyl, für jeweils gegebenenfalls durch Halogen, Nitro, Alkyl, Halogenalkyl, Alkoxy, Halogenalkoxy, Alkylthio, Alkylsulfinyl, Alkylsulfonyl, Dialkylaminosulfonyl und/oder Alkoxycarbonyl substituiertes Phenylsulfonyl oder Benzylsulfonyl steht,
$R^{11}$ für Hydroxy, für gegebenenfalls durch Alkoxycarbonyl substituiertes Alkoxy, Alkenyloxy, Benzyloxy, für jeweils gegebenenfalls durch Halogen, Cyano, Nitro, Alkyl, Halogenalkyl, Alkoxy, Halogenalkoxy, Alkylthio, Halogenalkylthio, Alkylsulfinyl, Alkylsulfonyl, Dialkylaminosulfonyl, Alkoxycarbonyl und/oder Alkylendioxy substituiertes Phenyl oder Naphthyl steht, oder $R^{11}$ weiterhin eine der oben für $R^{10}$ angegebenen Bedeutungen hat oder
$R^{11}$ zusammen mit $R^{10}$ für gegebenenfalls verzweigtes und gegebenenfalls durch Sauerstoff unterbrochenes Alkandiyl steht,
$R^{12}$ für Wasserstoff oder Alkyl steht,
$R^{13}$ für Alkyl, Cycloalkyl, Aralkyl, Alkoxy, Dialkylamino oder für gegebenenfalls durch Halogen, Alkyl und/oder Alkoxy substituiertes Phenyl steht
$R^{14}$ für jeweils gegebenenfalls durch Halogen substituiertes Alkyl oder Alkoxy steht,
$R^{15}$ für jeweils gegebenenfalls durch Halogen substituiertes Alkyl oder Alkoxy steht,
Q für Sauerstoff, Schwefel oder NH steht und
Z für Halogen, Cyano, Azido, Thiocyanato, Cyanamino, Hydrazino, Alkylhydrazino, Benzylhydrazino, gegebenenfalls durch Halogen, Alkyl, Halogenalkyl und/oder Alkoxy substituiertes Phenylhydrazino, für Alkylcarbonylhydrazino oder Alkoxycarbonylhydrazino, für gegebenenfalls durch Halogen, Nitro und/oder Alkyl substituiertes Benzoylhydrazino, für gegebenenfalls durch Halogen substituiertes Alkylsulfonylhydrazino, für gegebenenfalls durch Halogen, Cyano, Nitro, Alkyl, Halogenalkyl, Alkoxy, Halogenalkoxy, Alkylthio, Alkylsulfinyl, Alkylsulfonyl, Dialkylaminosulfonyl und/oder Alkoxycarbonyl substituiertes Phenylsulfonylhydrazino, für Alkylthio, Benzylthio, gegebenenfalls durch Halogen, Alkyl und/oder Alkoxy substituiertes Phenylthio, für Naphthylthio oder Alkoxycarbonylalkylthio oder für eine der nachstehenden Gruppierungen

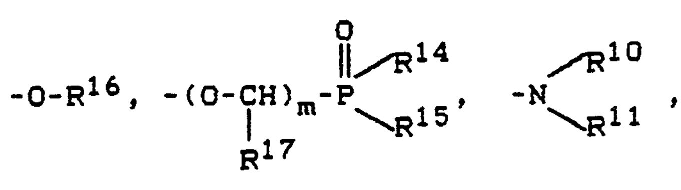

, , ,

$-O-(CH_2)_n-R^{18}$ steht, worin
m für die Zahlen 0 oder 1 steht,
n für die Zahlen 0, 1, oder 2 steht,
$R^{16}$ für Wasserstoff, für gegebenenfalls durch Halogen, Alkoxy, Alkylthio, Alkylsulfinyl, Alkylsulfonyl, Aralkoxy, Aralkylthio, Alkoxycarbonyl, Alkylaminocarbonyl, Azolyl oder Trialkylsilyl substituiertes Alkyl, für jeweils gegebenenfalls durch Halogen substituiertes Alkenyl oder Alkinyl, für jeweils gegebenenfalls durch

Halogen und/oder Alkyl substituiertes Cycloalkyl oder Cycloalkylalkyl, für gegebenenfalls durch Halogen, Alkyl, Alkoxy und/oder Alkoxycarbonyl substituiertes Benzyl, für jeweils gegebenenfalls durch Halogen, Alkyl, Halogenalkyl, Alkoxy und/oder Halogenalkoxy substituiertes Phenyl oder Naphthyl, oder für ein Ammonium-, Alkylammonium-, Alkalimetall- oder Erdalkalimetall-Äquivalent steht,

$R^{17}$ für Wasserstoff, Alkyl, jeweils gegebenenfalls durch Halogen, Alkyl, Halogenalkyl und/oder Alkoxy substituiertes Phenyl, Furyl, Thienyl oder Pyridyl steht,

$R^{18}$ für eine gegebenenfalls durch Halogen und/oder Alkyl substituierte heterocyclische Gruppierung aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxo perhydropyrrolyl, Pyridinyl oder Pyrimidinyl steht, sowie

$R^{10}$, $R^{11}$, $R^{14}$ und $R^{15}$ die oben angegebenen Bedeutungen haben,

sowie Salzen von Verbindungen der Formel (I), wobei die Verbindungen 2- Chlor-5-(2-chlor-4-trifluormethyl-phenoxy)-benzylchlorid und 2-Chlor-5-(2-chlor-4-trifluormethyl-phenoxy)-benzylcyanid und 2-Brom-5-(2-chlor-4-trifluormethyl-phenoxy)-benzylbromid ausgenommen sind,

dadurch gekennzeichnet, daß man

(a) halogensubstituierte Phenoxytoluol-Derivate der allgemeinen Formel (II)

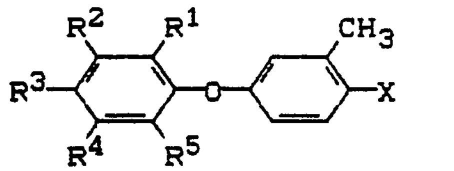

(II)

in welcher

$R^1$, $R^2$, $R^3$, $R^4$, $R^5$ und X die oben angegebenen Bedeutungen haben,

mit Halogenierungsmitteln, gegebenenfalls unter Belichtung, gegebenenfalls in Gegenwart von Katalysatoren und gegebenenfalls in Gegenwart von Verdünnungsmitteln umsetzt, oder daß man

(b) halogensubstituierte Phenoxybenzylhalogenide der allgemeinen Formel (Ia)

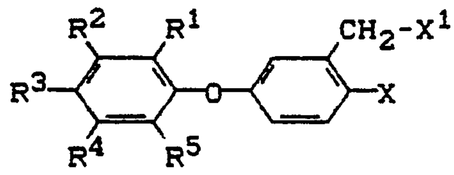

(Ia)

in welcher

$R^1$, $R^2$, $R^3$, $R^4$, $R^5$ und X die oben angegebenen Bedeutungen haben und

$X^1$ für Halogen steht,

mit nucleophilen Verbindungen der allgemeinen Formel (III)

M - Y (III)

in welcher

Y die oben angegebene Bedeutung hat und

M für Wasserstoff oder ein Metalläquivalent steht,

gegebenenfalls in Gegenwart eines Säureakzeptors und gegebenenfalls in Gegenwart eines Verdünnungsmittels umsetzt, oder daß man

(c) halogensubstituierte Phenoxybenzylhalogenide der allgemeinen Formel (Ia)

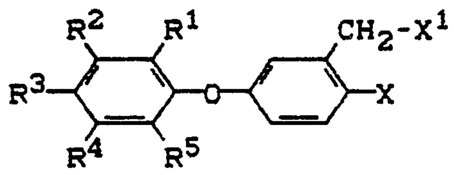

(Ia)

in welcher

$R^1$, $R^2$, $R^3$, $R^4$, $R^5$ und X die oben angegebenen Bedeutungen haben und

$X^1$ für Halogen steht,
mit Alkoxy-Phosphorverbindungen der allgemeinen Formel (IV)

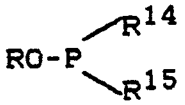

(IV)

in welcher
$R^{14}$ und $R^{15}$ die oben angegebenen Bedeutungen haben und
R für Niederalkyl steht,
gegebenenfalls in Gegenwart von Verdünnungsmitteln umsetzt, oder daß man

(d) halogensubstituierte Phenoxybenzylcyanide der allgemeinen Formel (Ib)

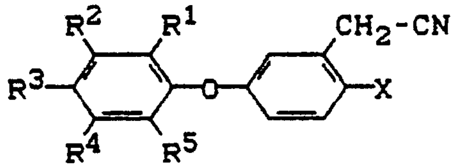

(Ib)

in welcher
$R^1$, $R^2$, $R^3$, $R^4$, $R^5$ und X die oben angegebenen Bedeutungen haben,
mit nucleophilen Verbindungen der allgemeinen Formel (V)
$M^1$ - Z (V)
in welcher
Z die oben angegebene Bedeutung hat und
$M^1$ für Wasserstoff oder ein Metalläquivalent steht,
gegebenenfalls in Gegenwart eines Katalysators und gegebenenfalls in Gegenwart einesVerdünnungsmittels umsetzt, oder daß man

(e) halogensubstituierte Phenoxbenzylverbindungen der allgemeinen Formel (Ic)

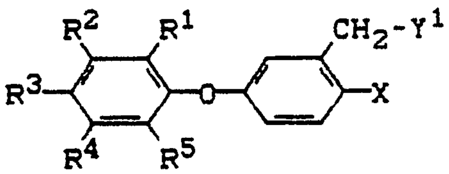

(Ic)

in welcher
$R^1$, $R^2$, $R^3$, $R^4$, $R^5$ und X die oben angegebenen Bedeutungen haben und
$Y^1$ für Cyano oder Alkoxycarbonyl steht,
mit Wasser, gegebenenfalls in Gegenwart eines Verseifungshilfsmittels und gegebenenfalls in Gegenwart eines organischen Lösungsmittels, umsetzt, oder daß man

(f) halogensubstituierte Phenoxyphenylessigsäuren der allgemeinen Formel (Id)

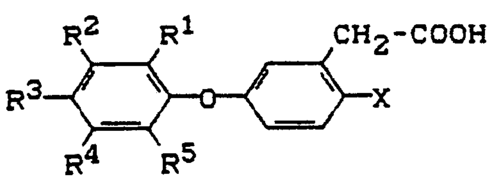

(Id)

in welcher
$R^1$, $R^2$, $R^3$, $R^4$, $R^5$ und X die oben angegebenen Bedeutungen haben,
mit Säurehalogeniden, gegebenenfalls in Gegenwart von Katalysatoren und gegebenenfalls in Gegenwart von Verdünnungsmitteln, umsetzt, oder daß man

(g) halogensubstituierte Phenoxyphenylessigsäurehalogenide der allgemeinen Formel (Ie)

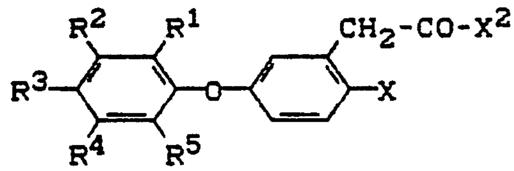

(Ie)

in welcher
$R^1$, $R^2$, $R^3$, $R^4$, $R^5$ und X die oben angegebenen Bedeutungen haben und
$X^2$ für Halogen steht
mit nucleophilen Verbindungen der allgemeinen Formel (V)
$M^1$ - Z (V)
in welcher
Z die angegebene Bedeutung hat und
$M^1$ für Wasserstoff oder ein Metalläquivalent steht,
gegebenenfalls in Gegenwart eines Säureakzeptors und gegebenenfalls in Gegenwart eines Verdünnungsmittels umsetzt, oder daß man

(h) halogensubstituierte Phenoxybenzylhalogenide der allgemeinen Formel (Ia)

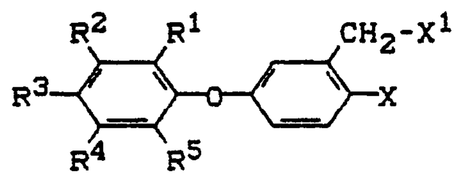

(Ia)

in welcher
$R^1$, $R^2$, $R^3$, $R^4$, $R^5$ und X die oben angegebenen Bedeutungen haben und
$X^1$ für Halogen steht,
mit Metallierungsmitteln und anschließend mit Carbonylverbindungen der allgemeinen Formel (VI)

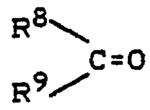

(VI)

in welcher
$R^8$ und $R^9$ die oben angegebenen Bedeutungen haben,
gegebenenfalls in Gegenwart eines Verdünnungsmittels umsetzt, oder daß man

(i) Phenoxybenzylverbindungen der allgemeinen Formel (VII)

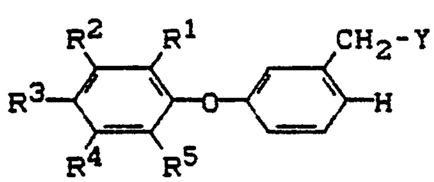

(VII)

in welcher
$R^1$, $R^2$, $R^3$, $R^4$, $R^5$ und Y die oben angegebenen Bedeutungen haben,
mit Halogenen, gegebenenfalls in Gegenwart von Katalysatoren und gegebenenfalls in Gegenwart von Verdünnungsmitteln umsetzt und
gegebenenfalls anschließend aus den Verbindungen der Formel (I) durch Umsetzung mit Säuren oder Basen Salze herstellt.

6. Herbizide Mittel, gekennzeichnet durch einen Gehalt an mindestens einer halogensubstituierten Phenoxybenzylverbindung der Formel (I) gemäß den Ansprüchen 1 bis 5.

7. Verfahren zur Bekämpfung von Unkräutern, dadurch gekennzeichnet, daß man halogensubstituierte Phenoxybenzylverbindungen der Formel (I) gemäß den Ansprüchen 1 bis 5 auf die Unkräuter und/oder ihren Lebensraum einwirken läßt.

8. Verwendung von halogensubstituierten Phenoxybenzylverbindungen der Formel (I) gemäß den Ansprüchen 1 bis 5 zur Bekämpfung von Unkräutern.

9. Verfahren zur Herstellung von herbiziden Mitteln, dadurch gekennzeichnet, daß man halogensubstituierte Phenoxybenzylverbindungen der Formel (I) gemäß den Ansprüchen 1 bis 5 mit Streckmitteln und/oder oberflächenaktiven Substanzen vermischt.